# EUROPÄISCHE PATENTSCHRIFT

(11) **EP 1 334 086 B1**
(45) Veröffentlichungstag und Bekanntmachung des Hinweises auf die Patenterteilung: **06.04.2005**
(21) Anmeldenummer: 01985700.2
(22) Anmeldetag: 13.09.2001
(51) Int. Cl.: C07C 309/65, C07C 311/08, C07C 311/09, A61K 31/18, A61K 31/255, A61P 25/28

(54) **PHENOXYPHENYL ALKANSULFONATE**
PHENOXYPHENYL ALKANE SULFONATES
SULFONATES D'ALCANE DE PHENOXYPHENYLE

(30) Priorität: 26.09.2000 DE 10047486
(43) Veröffentlichungstag der Anmeldung: 13.08.2003
(73) Patentinhaber: Bayer HealthCare AG, 51368 Leverkusen (DE)
(72) Erfinder: HEIL, Markus, 42799 Leichlingen (DE); MEIER, Heinrich, 42115 Wuppertal (DE); NAAB, Paul, 42287 Wuppertal (DE); VOERSTE, Arnd, 50677 Köln (DE); DE VRY, Jean-Marie-Viktor, 51503 Rösrath (DE); DENZER, Dirk, 42719 Solingen (DE); MAULER, Frank, 51491 Overath (DE); LUSTIG, Klemens, 42115 Wuppertal (DE); LENFERS, Jan-Bernd, 42327 Wuppertal (DE)
(86) Internationale Anmeldenummer: PCT/EP2001/010564
(87) Internationale Veröffentlichungsnummer: WO 2002/026702

(56) Entgegenhaltungen:
- WO-A-98/37061
- DE-A- 2 136 828
- DE-C- 216 642
- US-A- 3 914 418
- E.S. BLAKE: "Synthesis and properties of some m-bis[m-(perfluoroalkyl)phenoxy]benzenes" INDUSTRIAL AND ENGINEERING CHEMISTRY, PRODUCT RESEARCH AND DEVELOPMENT, Bd. 7, Nr. 2, 1968, Seiten 101-107, XP002190764 American Chemical Society, Washington, DC, US ISSN: 0196-4305
- J. VON SCHRAMM, ET AL.: "Nucleophile aromatische Substitution mit Aminophenolaten" LIEBIGS ANNALEN DER CHEMIE, Bd. 740, 1970, Seiten 169-179, XP000929254 Verlag Chemie, Weinheim, DE ISSN: 0170-2041
- F.J. MCEVOY, ET AL.: "2-Trifluoromethoxy- benz[b,e][1,4]diazepine and 2-trifluoro- methoxydibenz[b,f][1,4]oxazepine derivatives" JOURNAL OF MEDICINAL CHEMISTRY, Bd. 13, Nr. 3, März 1970 (1970-03), Seiten 295-297, XP000999698 American Chemical Society, Washington, DC, US ISSN: 0022-2623
- H. FUHRER, ET AL.: "A new ring closure reaction of 2-phenoxyphenols and 3-(phenoxy)pyridines. Synthesis of halogenated 10-methylphenoxines and 10-methyl[1,4]benzoxazino[3,2-b]pyridines" JOURNAL OF HETEROCYCLIC CHEMISTRY, Bd. 16, Nr. 6, September 1979 (1979-09), Seiten 1121-1134, XP002190765 Heterocorporation, Provo, US ISSN: 0022-152X

## Beschreibung

Die Erfindung betrifft neue Phenoxyphenyl Alkansulfonate, Verfahren zu ihrer Herstellung, ihre Verwendung zur Herstellung von Arzneimitteln zur Behandlung und/oder Prophylaxe von Krankheiten, insbesondere zur Behandlung von Schmerzzuständen und neurodegenerativen Erkrankungen.

Unter den Inhaltsstoffen der Hanfpflanze (Cannabis sativa) ist der pharmakologisch wichtigste das Δ⁹-Tetrahydrocannabinol (Δ⁹-THC), welches die wesentlichen Effekte von Cannabis auf das menschliche zentrale Nervensystem (ZNS) hervorruft. Potentielle historische und kontemporäre therapeutische Anwendungen von Cannabis-Präparaten umfassen u.a. Analgesie, Emesis, Anorexie, Glaukom und Bewegungsstörungen.

Bislang wurden zwei Subtypen von Cannabinoid-Rezeptoren und eine Spleiß-Variante identifiziert. CB1- und CB2-Rezeptoren besitzen sieben Transmembranregionen und gehören zur Familie der G-Protein-gekoppelten Rezeptoren. Der CB1-Rezeptor und die Spleiß-Variante CB1a sind überwiegend im Zentralen Nervensystem lokalisiert. Der CB2-Rezeptor wurde überwiegend im peripheren Gewebe, insbesondere in Leukozyten, Milz und Makrophagen gefunden.

Mehrere Strukturklassen von Cannabinoid-Rezeptor-Agonisten sind bisher bekannt: klassische Cannabinoide, wie beispielsweise Δ⁹-THC, nichtklassische Cannabinoide, wie beispielsweise Aminoalkylindole, und Eicosanoide. Zu den letzteren gehört der endogene CB1-Rezeptor-Agonist Anandamid.

In WO-A-98/37061, WO-A-00/10967 und WO-A-00/10968 werden bestimmte Aryloxyphenyl Alkansulfonate als Cannabinoid-Rezeptor-Agonisten zur Behandlung von neurodegenerativen Erkrankungen beschrieben.

Das US-A-3,462,473, *Biochem. Pharmacol.* **1972**, *21*, 1127-1134, *Fed. Proc. Fed*. *Amer. Soc. Exp. Biol.* **1971**, *30,* 841-847 und *J. Pharm. Sci.* **1973**, *62*, 1780-1784 offenbarten bestimmte p-Phenoxyphenyl Alkansulfonate und deren hypocholesterinämische bzw. hypolipidämische Wirkung.

Außerdem sind bestimmte Phenoxyphenyl Alkansulfonate und ihre Verwendung als Herbizide (1), antimikrobielle Mittel (2), Gleitmittel (3), Sensibilisatoren für Thermo-Papier (4) und synthetische Zwischenprodukte (5) bekannt: (1) EP-A-0 023 725; US-A-3,929,903; US-A-4,415,354; *Chem. Abstr.* **1979**, *91*, 175034 (JP-A-54066631); *Chem. Abstr.* **1981**, *94*, 156552 (JP-A-55154953); *Chem. Abstr.* **1981**, *95,* 168773 (JP-A-56046859); *Chem. Abstr.* **1981**, *95*, 168789 (JP-A-56079665); *Chem. Abstr.* **1989**, *111,* 2678 (JP-A-63104903); (2) DE-A-14 93 776; DE-A-21 31 754; US-A-3,629,477; US-A-3,772,445; US-A-3,850,972; CH-B-450 347; CH-B-459 656; *Chem. Abstr.* **1975,** *83*, 72725 (JP-B-50003375); (3) US-A-3,346,612; (4) US-A-4,837,197; (5) *Chem. Abstr.* **1997,** *127*, 26629 (JP-A-09087210); *Tetrahedr.* **1990**, *46.* 4161-4164, *J. Am. Chem. Soc.* **1998,** *39*, 435-436.

Aufgabe der vorliegenden Erfindung war es Cannabinoid-Rezeptor-Agonisten mit verbesserter Wirkung bereitzustellen.

Diese Aufgabe wird durch die erfindungsgemäßen, neuen Verbindungen gelöst.

Die vorliegende Erfindung betrifft daher neue Verbindungen der allgemeinen Formel (I), 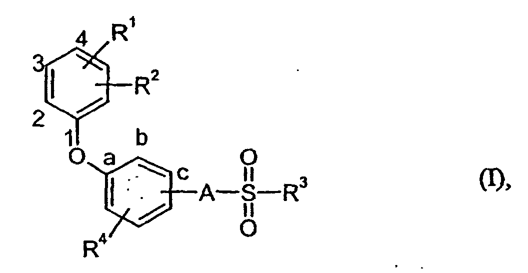 worin
- R¹: Wasserstoff, (C₁-C₄)-Alkyl, Halogen, Trifluormethyl, Trifluormethoxy, Cyano oder Nitro bedeutet,
- R²: Halogen, Trifluormethyl, Trifluonnethoxy, Cyano oder Nitro bedeutet,
- R³: (C₄-C₇)-Alkyl bedeutet, das ein- oder mehrfach durch Fluor oder Chlor substituiert sein kann,
- R⁴: Wasserstoff oder Halogen bedeutet, und
- A: Sauerstoff oder NH bedeutet.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze vorliegen. Im Allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Zur vorliegenden Erfindung gehören auch Ammoniumverbindungen, die durch Überführung der freien Amine mittels Alkylierung hergestellt werden können.

Im Rahmen der vorliegenden Erfindung haben die Substituenten im Allgemeinen die folgende Bedeutung:
(C₁-C₄)-Alkyl steht im Rahmen der Erfindung für einen geradkettigen oder verzweigten Alkylrest mit 1 bis 4 Kohlenstoffatomen. Beispielsweise seien genannt: Methyl, Ethyl, n-Propyl, Isopropyl, n-Butyl, i-Butyl, s-Butyl und t-Butyl.
(C₄-C₇)-Alkyl steht im Rahmen der Erfindung für einen geradkettigen oder verzweigten Alkylrest mit 4 bis 7 Kohlenstoffatomen. Beispielsweise seien genannt: n-Butyl, i-Butyl, s-Butyl, i-Butyl, t-Butyl, i-Pentyl, n-Pentyl, Hexyl, oder Heptyl. Bevorzugt sind n-Butyl, n-Pentyl und n-Hexyl.
Halogen schließt im Rahmen der Erfindung Fluor, Chlor, Brom und Iod ein. Bevorzugt sind Chlor oder Fluor.

Bevorzugt sind Verbindungen der allgemeinen Formel (I),
in welcher
- R¹: Wasserstoff, Fluor, Chlor, Methyl, Trifluormethyl, Trifluormethoxy, Cyano oder Nitro bedeutet,
- R²: Fluor, Trifluormethyl, Trifluormethoxy, Cyano oder Nitro bedeutet,
- R³: n-Butyl, n-Pentyl, 4,4,4-Trifluorbut-1-yl oder 5,5,5-Trifluorpent-1-yl bedeutet,
- R⁴: Wasserstoff bedeutet, und
- A: Sauerstoff bedeutet.

Besonders bevorzugt sind Verbindungen der allgemeinen Formel (I),
in welcher
R¹, R², R³, R⁴ und A die oben angegebene Bedeutung haben, und
ein Wasserstoffatom in Position 4 des mit R¹ und R² substituierten Phenylrings steht.

Dies kann durch folgendes Formelschema verdeutlicht werden:

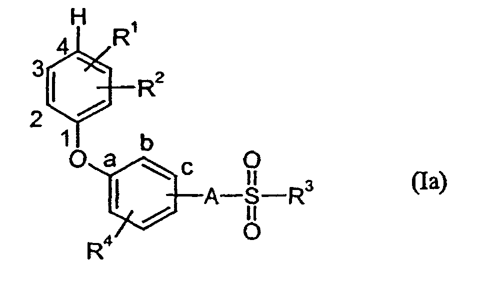

Ganz besonders bevorzugt sind Verbindungen der allgemeinen Formel (I),
in welcher
R¹, R², R³, R⁴ und A die oben angegebene Bedeutung haben, und
R¹ und R² die Positionen 2 und 3 des Phenylrings besetzen.

Die Positionen von R¹ und R² am Phenylring können durch folgendes Formelschema verdeutlicht werden:

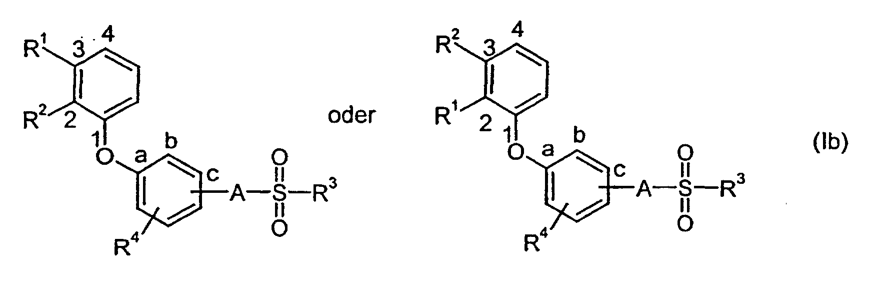

Ebenso ganz besonders bevorzugt sind Verbindungen der allgemeinen Formel (I),
in welcher
R¹, R², R³, R⁴ und A die oben angegebene Bedeutung haben, und
- A: in Position c des Benzolrests steht.

Die Position von A am Benzolrest kann durch folgendes Formelschema verdeutlicht werden:

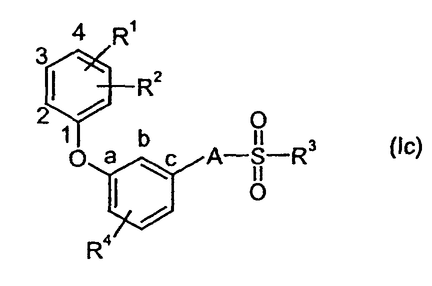

Ganz besonders bevorzugt sind Verbindungen der allgemeinen Formel (I),
in welcher
R¹, R², R³, R⁴ und A die oben angegebene Bedeutung haben,
R¹ und R² die Positionen 2 und 3 des Phenylrings besetzen, und
- A: in Position c des Benzolrests steht.

Die Positionen von R¹, R² und A können durch folgendes Formelschema verdeutlicht werden:

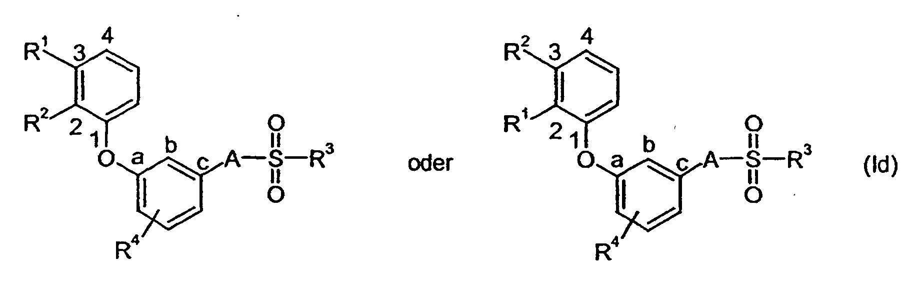

Außerdem wurde ein Verfahren zur Herstellung von Verbindungen der allgemeinen Formel (I) gefunden, dadurch gekennzeichnet, dass man eine Verbindung der allgemeinen Formel (II) 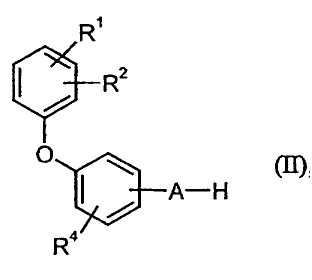 in welcher R¹, R², R⁴ und A die oben angegebene Bedeutung haben,
in einem inerten Lösemittel in Gegenwart einer geeigneten Base und gegebenenfalls in Gegenwart eines Phasentransfer-Katalysators mit einer Verbindung der allgemeinen Formel (III)

X¹-SO₂-R³ (III),

in welcher
- X¹: für eine geeignete Abgangsgruppe steht, und
- R³: die oben angegebene Bedeutung hat,
umsetzt.

Die Verbindungen der allgemeinen Formel (II) sind neu und können in Analogie zu allgemein bekannten Verfahren dadurch hergestellt werden, dass man eine Verbindung der allgemeinen Formel (IV) mit einer Verbindung der allgemeinen Formel (V) 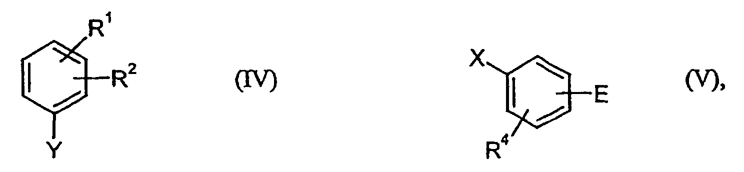 in welchen
R¹, R² und R⁴ die oben angegebene Bedeutung haben,
a) Y für eine Hydroxy-Gruppe und X für eine geeignete Abgangsgruppe
   oder umgekehrt
b) Y für eine geeignete Abgangsgruppe und X für eine Hydroxy-Gruppe,
   und
   - E: für eine Nitro-Gruppe oder eine Gruppe der Formel -O-R⁵ steht,
worin
- R⁵: für eine geeignete Hydroxy-Schutzgruppe steht,
in einem inerten Lösungsmittel in Gegenwart einer geeigneten Base und gegebenenfalls in Gegenwart einer Kupfer(I)- oder Kupfer(II)-Verbindung zunächst zu einer Verbindung der allgemeinen Formel (VI) 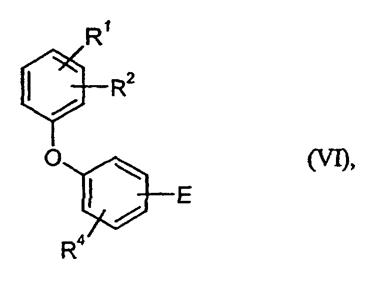 in welcher R¹, R², R⁴ und E die oben angegebene Bedeutung haben,
umsetzt,
und
[A] diese dann im Fall, dass E für eine Nitro-Gruppe steht, unter geeigneten Bedingungen nach üblichen Methoden zu einer Verbindung der allgemeinen Formel (IIa) 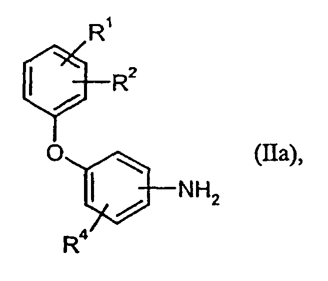 in welcher R¹, R² und R⁴ die oben angegebene Bedeutung haben,
   reduziert,
   oder
[B] im Fall, dass E für eine Gruppe der Formel -O-R⁵ steht, die Schutzgruppe R⁵ unter geeigneten Bedingungen nach üblichen Methoden unter Freisetzung einer Verbindung der allgemeinen Formel (IIb) 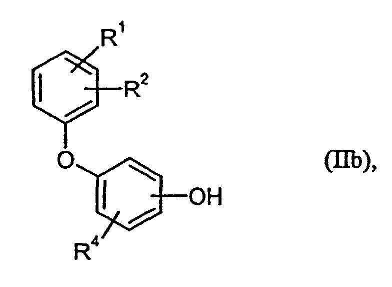
in welcher R¹, R² und R⁴ die oben angegebene Bedeutung haben,
entfernt.

Die Verbindungen der allgemeinen Formel (II) können auch hergestellt werden, indem man eine Verbindung der allgemeinen Formel (IV) mit einer Verbindung der allgemeinen Formel (VII) 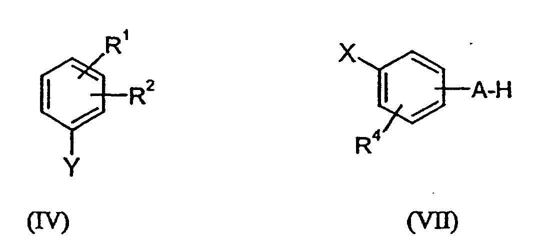 in welchen
R¹, R², R⁴, A, X und Y die oben angegebene Bedeutung haben,
in einem inerten Lösungsmittel in Gegenwart einer geeigneten Base und gegebenenfalls in Gegenwart einer Kupfer(I)- oder Kupfer(II)-Verbindung umsetzt.

Die erfindungsgemäßen Verfahren können durch das folgende Formelschema beispielhaft erläutert werden:

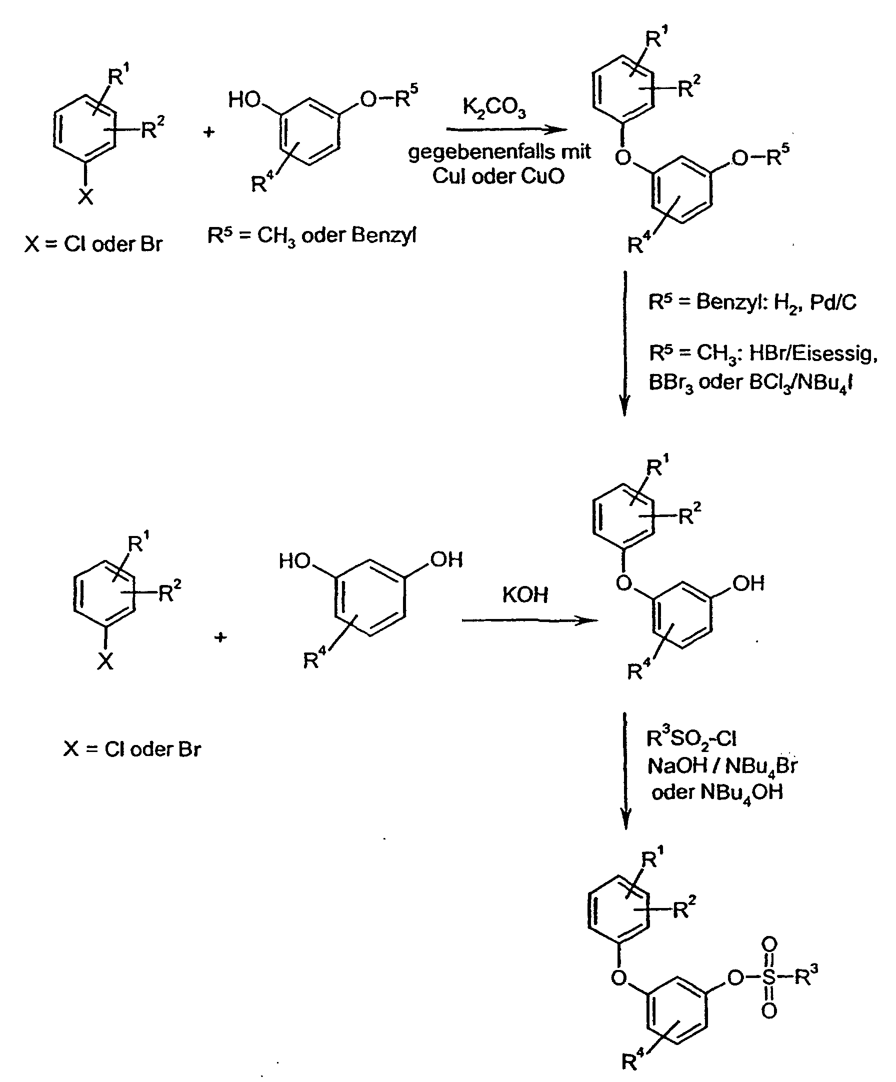

Inerte Lösungsmittel im Sinne der Erfindung sind solche Lösungsmittel, die sich unter den gewählten Reaktionsbedingungen nicht oder nur unwesentlich verändern.

Für das Verfahren (II) + (III) → (I) geeignete, inerte Lösungsmittel sind beispielsweise Ether, wie z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Ethylacetat, Pyridin, Triethylamin oder Picolin. Ebenso ist es möglich, Gemische der genannten Lösungsmittel, gegebenenfalls auch mit Wasser, zu verwenden. Besonders bevorzugt sind Methylenchlorid, Methylenchlorid/Wasser, Tetrahydrofuran, Dioxan und Dioxan/Wasser.

Als Basen eignen sich für Reaktion (II) + (III) → (I) organische Amine, insbesondere Tri-(C₁-C₆)-alkylamine wie beispielsweise Triethylamin oder Diisopropylethylamin, oder Hetero-cyclen wie Pyridin, Methylpiperidin, Piperidin oder N-Methylmorpholin, Alkali- bzw. Erdalkalimetallhydroxide oder -Carbonate, wie beispielsweise Natriumhydroxid, Kalium-hydroxid, Natriumcarbonat, Kaliumcarbonat oder Alkoholate, wie beispielsweise Natrium-methanolat oder Natriumethanolat. Bevorzugt sind Triethylamin, Pyridin und Natrium-hydroxid.

Die Basen werden im allgemeinen in einer Menge von 0,1 Mol bis 5 Mol, bevorzugt von 1 Mol bis 3 Mol jeweils bezogen auf 1 Mol der Verbindungen der allgemeinen Formel (II) eingesetzt.

Das Verfahren (II) + (III) → (I) kann gegebenenfalls auch in Gegenwart eines Phasentransferkatalysators durchgeführt werden. Als Phasentransferkatalysator eignen sich z.B. quartäre Ammoniumsalze, bevorzugt Tetrabutylammoniumbromid.

Als Abgangsgruppe X¹ eignet sich beispielsweise Halogen, vorzugsweise Chlor.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Das Verfahren (II) + (III) → (I) wird in einem Temperaturbereich von 0°C bis 100°C, vorzugsweise bei 0°C bis 30°C durchgerührt.

Als geeignete, inerte Lösungsmittel für die Verfahren (IV) + (V) → (VI) und (IV) + (VII) → (II) haben sich beispielsweise die folgenden erwiesen: organische Lösemittel wie Ether, wie z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylfbnnamid, N-Methyl-pyrrolidon, Hexamethylphosphorsäuretriamid, Ethylacetat, Pyridin, Triethylamin oder Picolin. Ebenso ist es möglich, Gemische der genannten Lösungsmittel, gegebenenfalls auch mit Wasser, zu verwenden. Besonders bevorzugt sind Pyridin, N-Methyl-pyrrolidon, Dimethylformamid und Dimethylsulfoxid.

Die Verfahren (IV) + (V) → (VI) und (IV) + (VII) → (II) können gegebenenfalls auch in Gegenwart einer Kupfer(I)- oder Kupfer(II)-Verbindung durchgeführt werden. Bevorzugt sind Kupfer(I)-iodid und Kupfer(II)-oxid.

Als Basen eignen sich für die Verfahren (IV) + (V) → (VI) und (IV) + (VII) → (II) Alkalimetallcarbonate und -hydrogencarbonate, insbesondere Natrium- und Kaliumcarbonat, Alkalimetallhydroxide, insbesondere Natriumhydroxid, oder organische Amine, insbesondere Tri-(C₁-C₆)-alkylamine, wie beispielsweise Triethylamin. Besonders bevorzugt sind Kaliumhydroxid, Natriumhydroxid und Kaliumcarbonat.

Die Basen werden im allgemeinen in einer Menge von 0,1 Mol bis 5 Mol, bevorzugt von 1 Mol bis 3 Mol jeweils bezogen auf 1 Mol der Verbindungen der allgemeinen Formel (IV) bzw. (V) eingesetzt.

Als Abgangsgruppe X beim Verfahren (IV) + (V) → (VI) Variante a) bzw. Y beim Verfahren (IV) + (V) → (VI) Variante b) eignet sich beispielsweise Halogen oder eine Sulfonato-Gruppe wie beispielsweise Triflat. Bevorzugt sind Fluor, Chlor oder Brom.

Die Umsetzungen können bei Normaldruck, aber auch bei erhöhtem oder erniedrigtem Druck (z.B. 0,5 bis 5 bar) durchgeführt werden. Im allgemeinen arbeitet man bei Normaldruck.

Die Reaktionen werden in einem Temperaturbereich von 20°C bis 200°C, vorzugsweise bei 100°C bis 160°C durchgeführt.

Methoden zur Reduktion einer aromatischen Nitro-Gruppe für den Verfahrensschritt (VI) → (IIa) sind bekannt (z.B. R. C. Larock, "Comprehensive Organic Transformations", New York, 1989, S. 411- 415 und die dort zitierte Literatur).

Die Einführung von Hydroxyschutzgruppen sowie Methoden zu deren Abspaltung sind bekannt (z.B. T.W. Greene, P.G.M. Wuts, "Protective Groups in Organic Synthesis", 2^{nd} Ed., New York, 1991 und die dort zitierte Literatur; *J.Org. Chem.* 1999, 64,9719-9721).

Als Schutzgruppe R⁵ bei der Reaktionssequenz (IV) + (V) → (VI) → (IIb) eignen sich beispielsweise Methyl, Benzyl, Allyl, Methoxymethyl, 2-Trimethylsilyl-ethoxymethyl oder Trimethylsilyl. Bevorzugt sind Methyl und Benzyl.

Die Verbindungen der allgemeinen Formel (III) sind kommerziell erhältlich, literaturbekannt oder können in Analogie zu literaturbekannten Verfahren synthetisiert werden (vgl. z.B. *J. Chem. Soc.* C 1968, 1265; *Chem. Ber.* 1967, 100, 1696; fluorierte Alkansulfonsäurechloride können z.B. erhalten werden nach WO-A-98/37061 oder DE-A-19 422 64).

Die Verbindungen der allgemeinen Formeln (IV), (V) und (VII) sind bekannt oder nach bekannten Verfahren herstellbar.

Die Verbindungen der allgemeinen Formeln (IV) und (V) sind kommerziell erhältlich, literaturbekannt oder können in Analogie zu literaturbekannten Verfahren hergestellt werden (vgl. z.B. J. March, "Advanced Organic Chemistry", 4^{th} Ed., Wiley, 1992, Seite 531-534 und 1295 bzw. die dort zitierte Literatur; *Synthesis* 1990, 1145-1147).

Überraschenderweise zeigen die erfindungsgemäßen Verbindungen ein nicht vorhersehbares, wertvolles pharmakologisches Wirkspektrum.

Sie zeichnen sich als hochwirksame Cannabinoid-Rezeptor-Agonisten mit hoher metabolischer Stabilität und hoher oraler Bioverfügbarkeit aus. Somit eignen sie sich besonders zur oralen Therapie.

Sie können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994) sowie neurodegenerativen Erkrankungen, insbesondere zur Behandlung von Krebs-induzierten Schmerzen und chronischen neuropathischen Schmerzen, wie zum Beispiel, bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Zur Kombination mit den erfindungsgemäßen Verbindungen zur Behandlung von akuten und/oder chronischen Schmerzen eignen sich beispielsweise Opiate, z.B. Tramadol, Morphin, Dihydrocodein, Dextropropoxyphen, Tricyclische Antidepressiva, z.B. Amitriptylin, Anticonvulsiva, z.B. Carbamazepin, Gabapentin, Nicht-steroidale Entzündungshemmer (NSAIDs), z.B. Aspirin, Ibuprofen, Naproxen, einschließlich COX-2-Inhibitoren, z.B. Rofecoxib, Celecoxib.

Gleichfalls eignen sich die erfindungsgemäßen Verbindungen auch zur Therapie von primären und/oder sekundären krankhaften Zuständen des Gehirnes, beispielsweise während oder nach cerebralen Vasospasmen, Migräne, Spastizität, Hypoxie und/oder Anoxie nicht vorher genannter Genese, perinataler Asphyxie, Autoimmunerkrankungen, Stoffwechsel- und Organerkrankungen, die mit einer Schädigung des Gehirnes einhergehen können sowie Schädigungen des Gehirnes infolge primärer Gehirnerkrankungen beispielsweise Krampfleiden und athero- und/oder arteriosklerotischer Veränderungen. Zur Behandlung chronischer oder psychiatrischer Leiden wie beispielsweise Depression, Magengeschwüren, neurodegenerativer Erkrankungen wie beispielsweise Alzheimerscher, Parkinsonscher oder Huntingtonscher Krankheit, Multipler Sklerose, amyotrophischer Lateralsklerose (ALS), Neurodegeneration durch akute und/oder chronische, virale oder bakterielle Infektionen und Multiinfarktdemenz sind die erfindungsgemäßen Verbindungen ebenfalls geeignet.

Darüber hinaus können sie in Arzneimitteln eingesetzt werden zur Behandlung von Emesis, Übelkeit, Glaukom, Asthma, Anorexie, Konvulsionen, Rheuma, Sedation und Bewegungsstörungen.

Die erfindungsgemäßen Substanzen eignen sich auch zur Behandlung von Erkrankungen, die durch bakterielle und/oder virale Infektion verursacht werden, die auf direkte und/oder indirekte Veränderungen des Immunsystems bzw. auf Fehlsteuerungen unter Mitwirkung des Immunsystems beruhen, wie z.B. bei lokalen oder systemischen Autoimmunerkrankungen (z.B. Lupus erythematodes in allen seinen Varianten), entzündlichen und/oder autoimmunologisch bedingten Erkrankungen der Gelenke (z.B. primär chronische Polyarthritis, traumatisch bedingten Entzündungen), entzündlichen und/oder autoimmunologisch bedingten Erkrankungen des Knochen- und Muskelapparates, entzündlichen und/oder autoimmunologisch bedingten krankhaften Prozessen der inneren Organe (z.B. Morbus Crohn, ulcerative Colitis, Glomerulonephritis) und der äußeren Organe (z.B. allergische Reaktionen durch aerogene Aufnahme von Antigenen) und des zentralen Nervensystems (z.B. Multiple Sklerose, Morbus Alzheimer, psychiatrische Erkrankungen) sowie der Sinnesorgane, primären und/oder sekundären und/oder autoimmunologischen Erkrankungen des blutbildenden Systems und des Immunsystems (z.B. Abstoßungsreaktionen, AIDS) selbst, sowie bei Hauterkrankungen entzündlicher und/oder immunologischer Genese bei Mensch und Tier. Ferner wirken diese Substanzen bei den indirekten Symptomen dieser Erkrankungen wie z.B. Schmerz.

Bevorzugt ist ihre Verwendung zur Behandlung von Schmerz, Spastizität, cerebralen Ischämien und Schädel/Hirn-Trauma.

Die in vitro-Wirkung der erfindungsgemäßen Verbindungen an Cannabinoid-Rezeptoren kann mit folgenden biologischen Assays gezeigt werden:

### 1. Ratten-CB1-Luciferase Reportergen Test

Stammkulturen einer Ratten-CHOCB1 Reporter-Zellinie wurden nach der in der WO-A-98/37061, S.55f. beschriebenen Methode hergestellt.

Für das Substanz-Screening wurde das folgende Testprotokoll verwendet: Die Stammkulturen wurden in 50 % Dulbecco's modifiziertem Eagle Medium / 50 % F-12 (DMEM/F12) mit 10 % FCS bei 37°C unter 10 % CO₂ gezüchtet und jeweils nach 2 bis 3 Tagen 1:10 gesplittet. Testkulturen wurden mit 5000 Zellen pro Napf in 96-well Platten ausgesät und 70 Stunden bei 37°C angezogen. Dann wurden die Kulturen vorsichtig mit Phosphat-gepufferter Saline gewaschen und mit serumfreiem Ultra-CHO Medium (Bio-Whittaker) rekonstituiert. Die in DMSO gelösten Substanzen wurden 1 x in Medium verdünnt und zu den Testkulturen pipettiert (maximale DMSO-Endkonzentration im Testansatz: 0,5 %). 20 Minuten später wurde Forskolin zugegeben und die Kulturen anschließend 3 Stunden im Brutschrank bei 37°C inkubiert. Danach wurden die Überstände entfernt und die Zellen durch Zugabe von 25 µl Lysereagens (25 mM Triphosphat, pH 7,8 mit 2mM DTT, 10 % Glycerin, 3 % TritonX100) lysiert. Direkt danach wurde Luciferase Substrat Lösung (2,5mM ATP, 0,5 mM Luciferin, 0,1 mM Coenzym A, 10mM Tricin, 1,35mM MgSO4, 15mM DTT, pH 7,8) zugegeben, kurz geschüttelt, und die Luciferase-Aktivität mit einem Hamamatsu Kamerasystem gemessen.

Zur Inaktivierung von Gᵢ-Proteinen wurden die Testkulturen vor dem Test für 16 Stunden mit 5 ng/ml (Endkonz.) Pertussis Toxin behandelt.

Die IC₅₀-Werte wurden mit dem Programm GraphPadPrism berechnet (Hill-Gleichung, speziell: one-site competition).

Beispiel 17 zeigt in diesem Test einen IC₅₀-Wert von 0,81 nM.

### 2. hCB2-Luciferase Reportergen Test

CHOluc9 Zellen wurden mit dem humanen CB2-Rezeptor stabil transfiziert. Transfektion, Klonselektion und Testentwicklung wurden analog zu den Arbeiten mit dem Ratten CB1-Rezeptor durchgeführt. Das folgende Testprotokoll wurde zur pharmakologischen Charakterisierung der Zellen und zur Substanz-Testung verwendet:

Die Stammkulturen wurden in 50 % Dulbecco's modifizierten Eagle Medium/50 % F-12 (DMEM/F12) mit 10 % FCS bei 37°C unter 10 % CO₂ gezüchtet und jeweils nach 2 bis 3 Tagen 1:10 gesplittet. Testkulturen wurden mit 5000 Zellen pro Napf in 96-well-Platten in DMEM/F12 Medium mit 5 % FCS ausgesät und 70 Stunden bei 37°C angezogen. Dann wurde das Medium von den Kulturen entfernt und durch serumfreies Ultra-CHO Medium (Bio-Whittaker) ersetzt. Die in DMSO gelösten Substanzen (200x Endkonzentration) wurden zu den Testkulturen pipettiert (maximale DMSO-Endkonz. im Testansatz: 0,5 %) und 20 min später wurde Forskolin zugegeben. Anschließend wurden die Kulturen 3,5 Stunden im Brutschrank bei 37°C inkubiert. Danach wurden die Überstände entfernt und die Zellen durch Zugabe von 25 µl Lysereagens (25 mM Trisphosphat, pH 7,8 mit 2 mM DTT, 10 % Glycerin, 3 % Triton X100) lysiert. Direkt anschließend wurden 50 µl Luciferase Substrat Lösung, doppelt konzentriert, (5 mM ATP, 1 mM, Luciferin, 0,2 mM Coenzym A, 10 mM Tricin, 1,35 mM MgSO₄, 15 mM DTT, pH 7,8) zugegeben, kurz geschüttelt, und die Luciferase-Aktivität mit einem Photomultiplier-Kamera-Messsystem (Hamamatsu) bestimmt.

Die IC₅₀-Werte wurden mit dem Program GraphPad Prism™ berechnet (Hill-Gleichung; speziell: one site competition).

### 3. Bindung an Ratten Cortex Membranen

Membranprotein wird nach Standardmethoden aus unterschiedlichen Geweben bzw. von Zellen präpariert. Puffer, markierter Ligand, DMSO oder Teststubstanz werden zusammenpipettiert, anschließend werden 100 µg Protein hinzugegeben, die Mischung gut vermischt und 60 min bei 30°C im Wasserbad inkubiert. Nach Ablauf der Inkubationszeit wird die Reaktion durch Zugabe von eiskaltem Inkubationspuffer in jedes Röhrchen gestoppt. Nach Abfiltrieren wird mit 3/4 ml Inkubationspuffer nachgewaschen. Die Filter werden in Minivials überführt, die Radioaktivität wird in einem Flüssigszintillationszähler bestimmt.

Die metabolische Stabilität der erfindungsgemäßen Verbindungen kann in folgendem *in vitro*-Assay gezeigt werden:

### 4. Mikrosomale Stabilitätsuntersuchungen

Die metabolische Stabilität der erfindungsgemäßen Verbindungen kann in Lebermikrosomen der Ratte gemessen werden (analog *J*. *Pharmacol. Exp. Ther.* **1997**, *283.* 46-58).

Zur Bestimmung der mikrosomalen Stabilität und Hochrechnung der auf Grund des Leber-First-Pass Effektes (Phase 1 Reaktionen) maximal möglichen Bioverfügbarkeit (Fmax) wird die Substanz in geringer Konzentration mit mikrosomalem Protein über 15 Minuten unter Zusatz von Cofaktoren bei 37°C inkubiert.

Die Inkubation, sowie die Probenabnahme erfolgt auf einem modifizierten Pipettierautomaten der Firma Canberra Packard.

Wie der Vergleich mit einem Beispiel aus der WO-A-98/37061 gezeigt, sind die erfindungsgemäßen Verbindungen in diesem Test metabolisch stabiler:

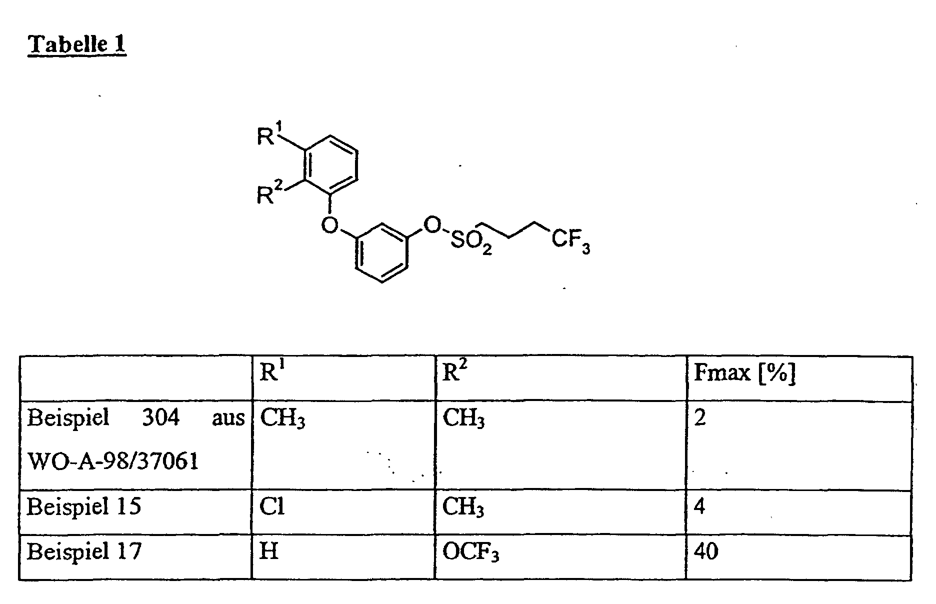

Die Bioverfügbarkeit der erfindungsgemäßen Verbindungen sowie weitere pharmakokinetische Parameter können *in vivo* in folgender Weise bestimmt werden:

### 5. Pharmakokinetik in der Ratte

### a) Intravenöse Infusion

Die Substanz wird durch eine Braunüle über eine laterale Schwanzvene direkt in den Blutstrom über 15 Minuten infundiert. Für die exakte Verabreichung der gewählten Dosis und des Volumens wird eine kalibrierte 20 ml Spritze verwendet. Für die Infusion wird eine Pumpe von Braun Melsungen Nr.152440/1 verwendet.

### b) Perorale Applikation

Die Substanzgabe erfolgt als Bolus über eine Schlundsonde.

### c) Probennahme und Aufarbeitung

### Blut und Plasma

Blutproben werden von katheterisierten Tieren (Vena jugularis) in heparinisierten Röhrchen gesammelt. Das Blut wird zentrifugiert und das Plasma auf geeignete Weise für die Analytik (LC-MS-MS) vorbereitet. Das Plasma wird bis zur Analytik bei <-15 °C aufbewahrt.

### d) Pharmakokinetische Ergebnisse

Microsomale Daten (Lebermikrosomen der Ratte) sagen eine maximal mögliche Verfügbarkeit von bis zu 100 % voraus.

Die aus den in vivo-Versuchen (Ratte) abgeleiteten pharmakokinetischen Parameter für Beispiel 22 sind:
- p.o.-Daten:: **(Dosis: 3mg/kg):** AUCₙₒᵣₘ: 0,102 kg*h/l, C_{max,norm}: 0,0198 kg/l, tₘₐₓ: 2,29 h, t_{½}: 2,36h, F: 33%.
- **i.v.-Daten:**: (Dosis: 0,3mg/kg): AUCₙₒᵣₘ: 0,307 kg*h/l, C_{max,norm}: 0,5978 kg/l, Vₛₛ: 4,12/kg, t_{½}: 1,6 h.

Hierbei bedeuten:
- AUCₙₒᵣₘ:: die auf 1 mg/kg Dosis normierte Fläche unter der Plasmakonzentration-Zeit-Kurve;
- C_{max,norm}:: die auf 1 mg/kg Dosis normierte maximale Plasmakonzentration nach einmaliger Applikation;
- tₘₐₓ:: die Zeit, zu der die Maximalkonzentration im Plasma nach Einmalgabe erreicht wird;
- t_{½}:: terminale Halbwertszeit;
- F:: Bioverfügbarkeit; hier Anteil der Dosis in Prozent, der im Vergleich zu einer i.v.-Applikation systemisch verfügbar ist;
- Vₛₛ:: apparentes Verteilungsvolumen im "steady state".

Die *in vivo*-Wirkung der erfindungsgemäßen Verbindungen kann beispielsweise in folgenden Tiermodellen gezeigt werden:

### 6. Hypothermie (Ratte)

Die *in vivo*-agonistische Wirkung am CB1 Rezeptor wurde überprüft im Hypothermie Assay der Ratte.

Fünf Minuten nach Bestimmung der Basal-Körpertemperatur via Oesophagus Temperatursonde wird die Prüfsubstanz (p.o.) appliziert. Eine Kontrollgruppe erhält, ebenfalls p.o., nur das Lösungsmittel der Prüfsubstanzen (Cremophore EL 1-10 % + Aqua Dest.). Die Körpertemperatur wird 120 und 240 Minuten nach p.o.-Applikation gemessen. Die Gruppengröße pro Dosis beträgt 5-7 Tiere (Ratten).

| Ratten Hypothermie - Agonismus Prüfung | |
|---|---|
| **Beispiel** | **ED**_{**-1°C**}^{**a**}**) [mg/kg]** |
| 22 | 10 |

| | |
|---|---|
| ^{a)} Effektive Dosis für die Reduktion der Körpertemperatur um 1°C | |

Die Eignung der erfindungsgemäßen Verbindungen zur Behandlung von Schmerzzuständen kann in folgenden Tiermodellen gezeigt werden:

### 7. Axotomie von Ischiasverzweigungen bei der Ratte (Chronisches Schmerzmodell)

Unter Pentobarbital-Anästhesie wird die Trifurkation eines Ischias-Nerven freipräpariert, der Peroneus- sowie der Tibialiszweig werden axotomiert nachdem die Nerven proximal der Axotomiestelle ligiert wurden. Kontrolltiere erhalten eine Scheinoperation. Nach der Operation entwickeln die axotomierten Tiere eine chronische mechanische Hyperalgesie. Diese Hyperalgesie wird mit Hilfe eines Druckaufnehmers im Vergleich zu scheinoperierten Tieren getestet (elektronisches von Frey Anästhesiometer, IITC Inc.-Life Science Instruments, Woodland Hills, CA, USA).

Die Substanzapplikation erfolgt zu unterschiedlichen Zeitpunkten vor der Schmerztestung über unterschiedlichen Applikationsrouten (i.v., i.p., p.o., i.t., i.c.v., transdermal).

Beispiel 22 reduziert die Hyperalgesie im Modell bei einer minimal wirksamen Dosierung von 1 mg/kg, p.o. (akute Applikation, 60 Minuten vor Test).

Die Eignung der erfindungsgemäßen Verbindungen beispielsweise zur Behandlung von neurodegenerativen Erkrankungen kann im Modell der Permanenten focalen cerebralen Ischämie bei der Ratte (MCA-O) oder im Modell des Subduralen Hämatoms bei der Ratte (SDH) gezeigt werden (WO-A-98/37061, S.60f).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 1 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Bestimmung der Retentionszeit von Ausgangsverbindungen und Herstellungsbeispielen mit HPLC erfolgte unter folgenden Bedingungen:

Säule: Kromasil C18 60*2; Injektionsvolumen 1,00 µl; Fluss: 0,75 ml/min; Eluent: A= 0,01M aq H₃PO₄, B= CH₃CN; Gradient [t(min): A/B)]: 0: 90/10; 0,5: 90/10; 4,5: 10/90; 6,5: 10/90; 7,5: 90/10.

### Abkürzungen

- aq.: wässrig
- CH: Cyclohexan
- DC: Dünnschichtchromatographie
- DCI: direkte chemische Ionisation (bei MS)
- DCM: Dichlormethan
- EE: Ethylacetat (Essigsäureethylester)
- EI: Elektronenstoß-Ionisation (bei MS)
- ESI: Elektrospray-Ionisation (bei MS)
- HPLC: Hochdruck-, Hochleistungsflüssigchromatographie
- Me: Methyl
- MG: Molekulargewicht
- MS: Massenspektroskopie
- NMR: Kernresonanzspektroskopie
- R_{f}: Retentionsindex (bei DC)
- Rₜ: Retentionszeit (bei HPLC)

### Ausgangsverbindungen

### Beispiel I

### 3-Methoxy-1-(3-methyl-2-nitrophenoxy)benzol

### (Diphenylethersynthese - Methode A)

14,7 g (96,0 mmol) 3-Methyl-2-nitrophenol, 53,9 g (288 mmol) 3-Bromanisol und 18,3 g (96,0 mmol) Kaliumcarbonat werden in etwa 600 ml Pyridin vorgelegt und auf etwa 140°C erhitzt. Man läßt leicht wieder abkühlen und gibt 18,3 g (96 mmol) Kupfer(I)-iodid zu. Der Ansatz wird ca. 60 h bei etwa 140°C gerührt. Nach Entfernen des Lösungsmittels im Vakuum nimmt man den Rückstand in Toluol auf und engt nochmals ein. Der Rückstand wird in Dichlormethan aufgenommen und über Kieselgur filtriert. Nach Waschen mit weiterem Dichlormethan wird nacheinander mit 5N HCl, 2N NaOH, 5N HCl, Wasser und Kochsalzlösung gewaschen. Nach Trocknen über Magnesiumsulfat wird im Vakuum eingeengt und das erhaltene Rohprodukt durch Kugelrohrdestillation gereinigt.
Ausbeute: 3,50 g (13 %; HPLC-Reinheit 94%)
R_{f}: 0,28 (Cyclohexan/Ethylacetat 5:1)
MS (EI): 259 (100%, [M]⁺)
HPLC: Retentionszeit = 4,94 min
¹H-NMR (300 MHz, CDCl₃): δ /ppm = 2,37 (s, 3H), 3,78 (s, 3H), 6,1-6,65 (m, 2H), 6,67-6,74 (m, 1H), 6,83 (d, J=8Hz, 1H), 6,99 (d, J=8Hz, 1H), 7,14-7,32 (m, 2 H).

### Beispiel II

### 3-Methoxy-1-[2-(trifluormethyl)phenoxy]benzol

### (Diphenylethersynthese - Methode B)

50,0 g (222 mmol) 2-Brombenzotrifluorid, 27,6 g (222 mmol) 3-Methoxyphenol und 30,7 g (222 mmol) Kaliumcarbonat werden in etwa 450 ml Pyridin vorgelegt und kurz auf etwa 100°C erhitzt. Man läßt leicht wieder abkühlen und gibt 17,7 g (222 mmol) Kupfer(II)oxid zu. Der Ansatz wird ca. 48 h unter Rückfluß (Badtemperatur etwa 140°C) gerührt. Nach Entfernen des Lösungsmittels im Vakuum nimmt man den Rückstand in Dichlonnethan auf und schüttelt mit 2N Salzsäure aus. Anschließend wird die organische Phase mit 1N Natronlauge und Wasser gewaschen. Nach Trocknen über Magnesiumsulfat wird im Vakuum eingeengt und das erhaltene Rohprodukt durch Kugelrohrdestillation gereinigt.
Ausbeute: 37,2 g (62 %; HPLC-Reinheit 98%)
R_{f}: 0,47 (Cyclohexan/Ethylacetat 5:1)
MS (EI): 268 (100%, [M]⁺)
HPLC: Retentionszeit = 5,14 min
¹H-NMR (200 MHz, CDCl₃): δ /ppm = 3,79 (s, 3H), 6,56-6,65 (m, 2 H), 6,71 (ddd, J=8Hz, 2 Hz, 1Hz, 1H), 6,97 (d, J=8Hz, 1H), 7,17 (t, J=8Hz, 1H) 7,25 (t, J=8Hz, 1H), 7,46 (td, J= 8Hz, 1Hz, 1H), 7,66 (d, J=8 Hz, 1H).

### Beispiel III

### 1-[2-Cyano-3-(trifluormethyl)phenoxy]-3-methoxybenzol

### (Diphenylethersynthese - Methode C)

Unter Argon werden 10,7 g (52,1 mmol) 2-Chlor-6-(trifluormethyl)benzonitril [Beispiel 5 in DE-A-38 36 159; 2-Chlor-6-(trichloromethyl)benzonitril herstellbar aus 2,6-Dimethyl-benzonitril nach Beispiel 3 in DE-A-2 214 058] in wasserfreiem DMF vorgelegt, mit 7,19 g (52,1 mmol) Kaliumcarbonat und 6,46 g (52,1 mmol) 3-Methoxyphenol versetzt und 5h bei 100°C gerührt. Anschließend wird mit 500 ml 2N NaOH und 200 ml gesättigter Kochsalzlösung versetzt. Man extrahiert zweimal mit ca. 300 ml Ether, trocknet die vereinigten organischen Phasen über Magnesiumsulfat, dampft im Vakuum ein, und flashchromatographiert an 450 g Kieselgel im Laufinittel Toluol. Produktfraktionen werden zur Trockene eingedampft und zum verbleibenden Öl wird wenig Ether gegeben. Man läßt auskristallisieren, saugt ab und wäscht mit Pentan nach.
Ausbeute: 9,36 g (57%; HPLC-Reinheit 96%)
R_{f}: 0,39 (Toluol)
Schmelzpunkt: 68°C
HPLC: Retentionszeit = 4,89 min
¹H-NMR (200 MHz, CDCl₃): δ /ppm = 3,72 (s, 3H), 6,62-6,87 (m, 3H), 7,08 (d, J=8Hz, 1H), 7,33 (t, J=8Hz, 1H), 7,44 (d, J=8Hz, 1H), 7,57 (t, J=8Hz, 1H).

### Beispiel IV

### 1-[2-Chlor-3-(trifluormethyl)phenoxy]-3-nitrobenzol

### (Diphenylethersynthese - Methode D)

Unter Argon werden 1,00 g (5,09 mmol) 2-Chor-3-(trifluormethyl)phenol in 10 ml DMF vorgelegt, mit 0,72 g (5,09 mmol) 3-Fluornitrobenzol und 0,70 g (5,09 mmol) Kaliumcarbonat versetzt. Das Gemisch wird für ca. 16 h zum Rückfluß erhitzt. Nach Abkühlen gibt man den Ansatz auf 50 ml 2N Natronlauge und rührt eine Stunde nach, dann gibt man 20 ml Natriumchloridlösung zu und rührt weitere 30 Minuten. Anschließend wird mit Dichlormethan extrahiert, die organische Phase über Magnesiumsulfat getrocknet und im Vakuum eingeengt. Reinigung durch Chromatographie an Kieselgel im Laufmittel Cyclohexan/Ethylacetat 20:1 ergibt 0,69 g (42 %, HPLC-Reinheit: 100%) der Zielverbindung.
R_{f}: 0,39 (Cyclohexan/Ethylacetat 2:1)
MS (EI): 317(100%, [M]⁺)
HPLC: Retentionszeit = 5,22 min
¹H-NMR (300 MHz, DMSO-d₆): δ /ppm = 7,51 (dd, J=8Hz, 2Hz, 1H), 7,56-7,83 (m, 5H), 8,05 (dd, J=8Hz, 2Hz, 1H).

Die folgenden Beispiele V - XIII werden auf analoge Weise entsprechend den Ausgangsverbindungs-Verfahrensmethoden A oder B aus den entsprechenden Ausgangsverbindungen hergestellt:

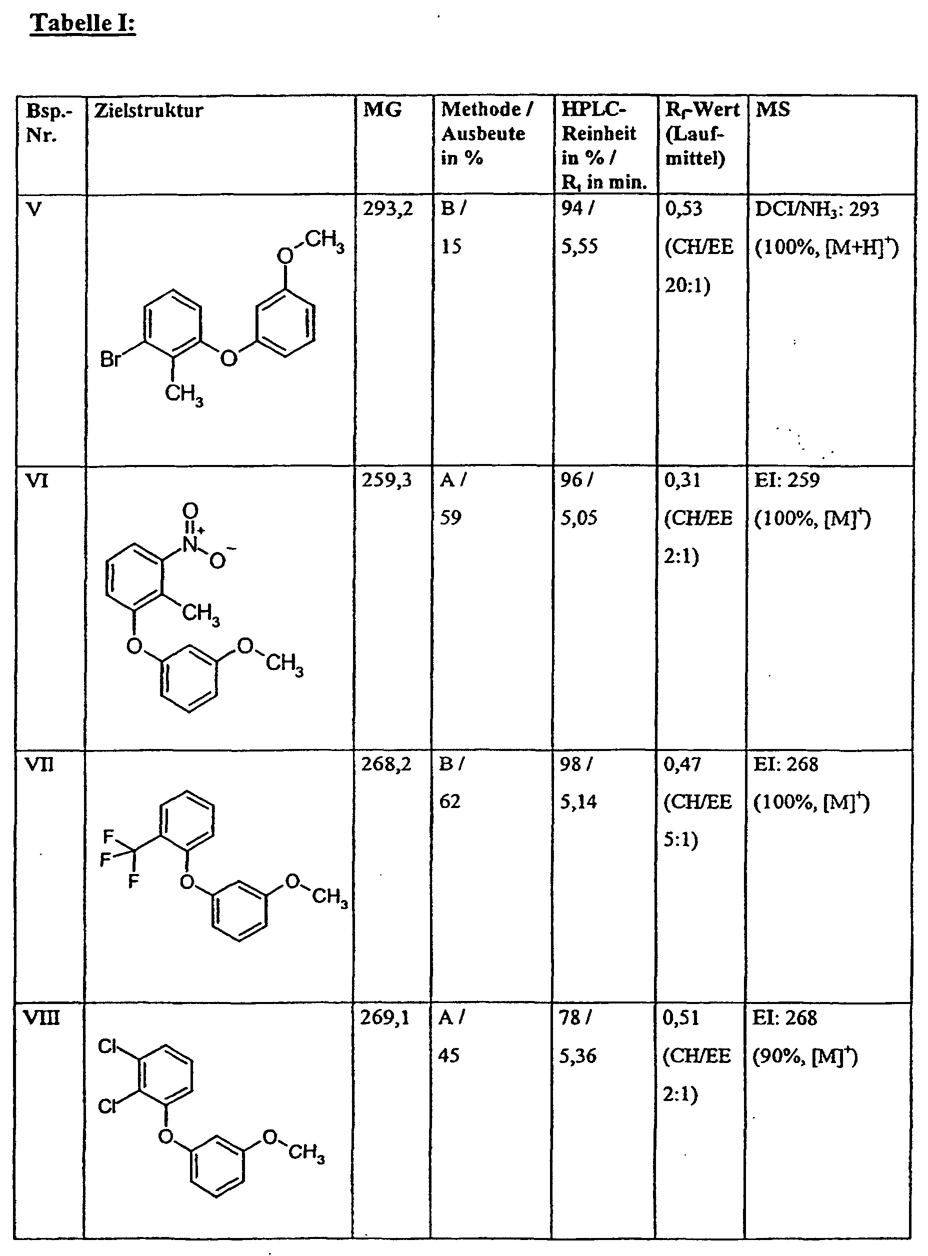

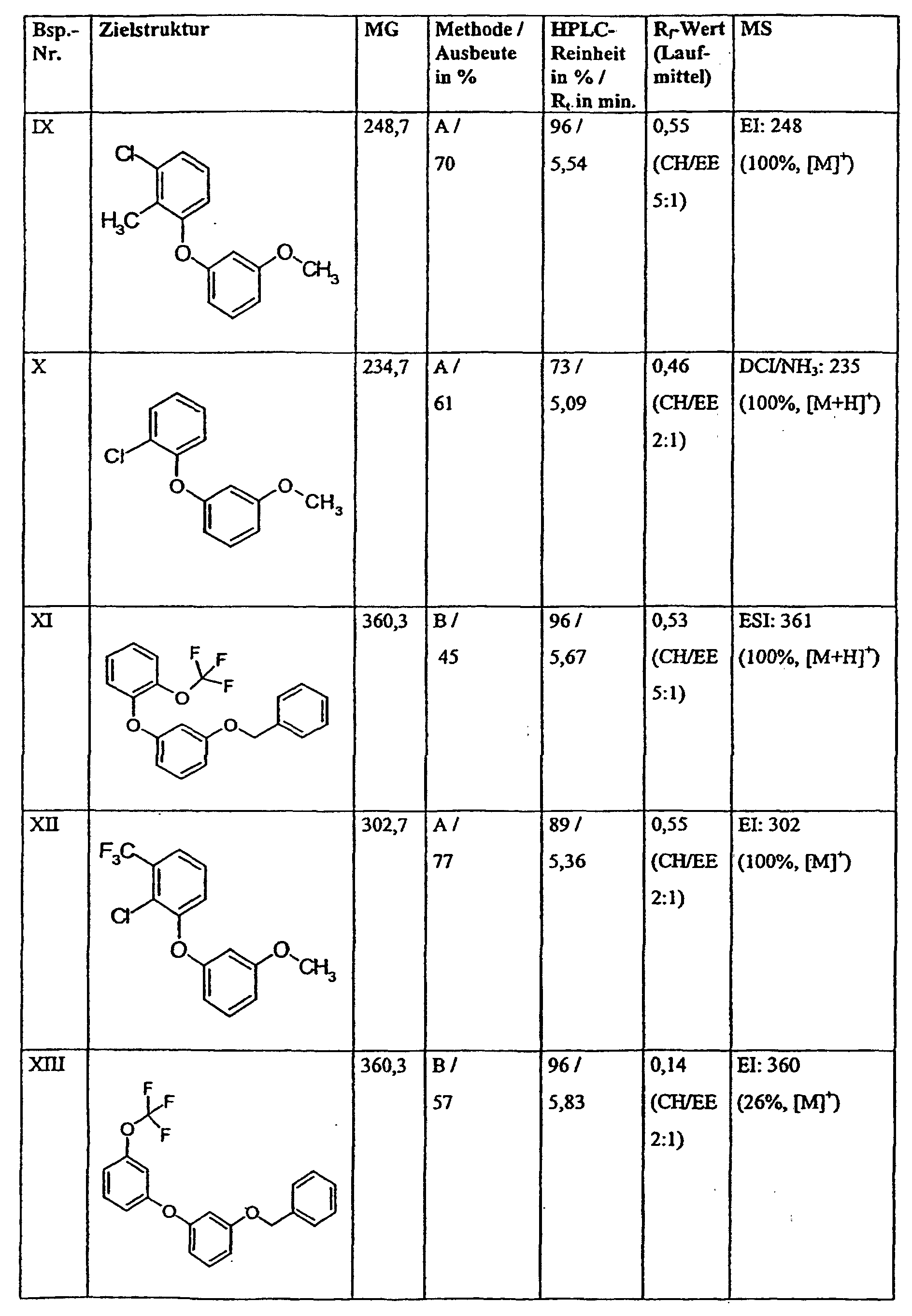

### Beispiel XIV

### 3-(3-Methyl-2-nitrophenoxy)phenol

### (Methyletherspaltung - Methode A)

Unter Argon werden 500 mg (1,93 mmol) 1-Methoxy-3-(3-methyl-2-nitrophenoxy)benzol in 2 ml wasserfreiem Dichlormethan vorgelegt und die Lösung auf -20°C abgekühlt. Bei dieser Temperatur werden 5,8 ml einer 1M Lösung von Bortribromid in Dichlormethan zugegeben. Man läßt auf 0°C kommen und rührt für 1h. Nach Versetzen mit Wasser wird dreimal mit Dichlormethan extrahiert. Die vereinigten organischen Phasen werden mit Natriumhydrogencarbonatlösung gewaschen, über Magnesiumsulfat getrocknet und im Vakuum eingeengt. Chromatographische Reinigung an Kieselgel im Laufmittel Cyclohexan/Ethylacetat 30:1 ergibt 424 mg (89%) der Zielverbindung.
R_{f}: 0,18 (Cyclohexan/Ethylacetat 2:1)
MS (EI): 245 ([M]⁺)
HPLC: Retentionszeit = 4,40 min
¹H-NMR (300 MHz, CDCl₃): δ /ppm = 2,37 (s, 3H), 4,88 (breites s, 1H), 6,54 (t, J=2Hz, 1H), 6,57-6,66 (m, 2H), 6,85 (d, J=8Hz, 1H), 7,01 (d, J=8Hz, 1H), 7,19 (t, J=8Hz, 1H), 7,27 (t, J=8Hz, 1H).

### Beispiel XV

### 3-[2-Cyano-3-(trifluormethyl)phenoxy]-phenol

### (Methyletherspaltung - Methode B)

Unter Argon werden 10,0 g (34,1 mmol) 1-(2-Cyano-3-trifluormethylphenoxy)-3-methoxybenzol in wasserfreiem Dichlormethan vorgelegt und mit 13,9 g (37,5 mmol) n-Tetrabutylammoniumiodid versetzt. Man kühlt auf -78°C ab und tropft langsam 120 ml einer 1N Lösung von Bortrichlorid in Dichlormethan zu, wobei man die Temperatur nicht über -70°C kommen läßt. Innerhalb von 2h läßt man auf RT aufwärmen. Die Reaktionsmischung wird auf 300 ml Eiswasser gegossen, die Mischung dreimal mit Dichlormethan extrahiert, die organische Phase 2 x mit gesättigter Natriumhydrogencarbonatlösung und 1 x mit Kochsalzlösung gewaschen. Man trocknet über Magnesiumsulfat und flashchromatographiert an ca. 400 g Kieselgel mit Dichlormethan. Zum öligen erhaltenen Produkt gibt man Pentan und läßt auskristallisieren.
Ausbeute: 7,75 g (96%; HPLC-Reinheit 96%)
R_{f}: 0,16 (CH₂Cl₂)
Schmelzpunkt: 108°C
HPLC: Retentionszeit = 4,41 min
¹H-NMR (300 MHz, CDCl₃): δ /ppm = 5,13 (s, 1H), 6,59-6,78 (m, 3H), 7,11 (d, J=8 Hz, 1H), 7,28 (t, J=8Hz, 1H), 7,45 (d, J=8 Hz, 1H), 7,58 (t, J=8 Hz, 1H).

### Beispiel XVI

### 3-[2-Chlor-3-(trifluormethyl)phenoxy]phenol

### (Methyletherspaltung -Methode C)

600 mg (1,98 mmol) 3-Methoxy-1-[2-chlor-3-(trifluormethyl)phenoxy]-benzol werden in 6 ml Essigsäure vorgelegt, mit 3,60 ml 48%iger wäßriger Bromwasserstoffsäure versetzt und für 4h zum Rückfluß erhitzt. Nach Abkühlen wird mit Wasser verdünnt und mit Ethylacetat extrahiert. Die organischen Phasen werden dreimal mit Wasser gewaschen, dann über Magnesiumsulfat getrocknet und im Vakuum eingeengt. Chromatographie an Kieselgel im Laufinittel Dichlormethan/Cyclohexan 2:1 ergibt 484 mg (81%, HPLC-Reinheit 96%) der Zielverbindung.
R_{f}: 0,39 (Cyclohexan/Ethylacetat 2:1)
MS (EI): 288 ([M]⁺)
HPLC: Retentionszeit = 4,80 min
¹H-NMR (300 MHz, DMSO-d₆): δ/ppm = 6,37 (t, J=2Hz), 6,44 (ddd, J=8Hz, 2Hz, 1Hz, 1H), 6,59 (ddd, J=8Hz, 2Hz, 1Hz, 1H), 7,20 (t, J=8Hz, 1H), 7,39 (dd, J=8Hz, 1Hz, 1H), 7,56 (t, J=8Hz, 1H), 7,68 (dd, J= 8Hz, 1Hz, 1H), 9,69 (s, 1H).

### Beispiel XVII

### 3-[3-(Trifluormethyl)phenoxy]phenol

### (Benzyletherspaltung - Methode D)

1,70 g (4,72 mmol) 3-Benzyloxy-1-[3-(trifluormethyl)phenoxy]-benzol werden in einem Hydriergefäß in 135 ml Tetrahydrofuran und 15 ml Ethanol suspendiert und nach Zugabe von 170 mg Pd 10%ig auf Kohle bei Normaltemperatur unter 1 atm Wasserstoff über Nacht hydriert. Zur Aufarbeitung wird der Katalysator über Kieselgur abfiltriert, das Filtrat eingeengt und über 130 g Kieselgel in einem Cyclohexan/Ethylacetat-Gradienten 10:1 bis 1:1 flashchromatographiert.

Entfernen des Lösungsmittels ergibt 1,27 g (99%, HPLC-Reinheit 95%) der Zielverbindung.
R_{f}: 0,28 (Cyclohexan/Ethylacetat 5:1)
MS (EI): 270 ([M]⁺)
HPLC: Retentionszeit = 4,78 min
¹H-NMR (200 MHz, CDCl₃): δ /ppm = 4,97 (s, 1H), 6,53 (t, J=2Hz, 1H), 6,56-6,67 (m, 2H), 6,85-7,01 (m, 3H), 7,22 (t, J= 8Hz, 1H), 7,34 (t, J=8 Hz, 1H).

Die folgenden Beispiele XVIII - XXV werden auf analoge Weise entsprechend den Verfahrensmethoden A, C oder D hergestellt:

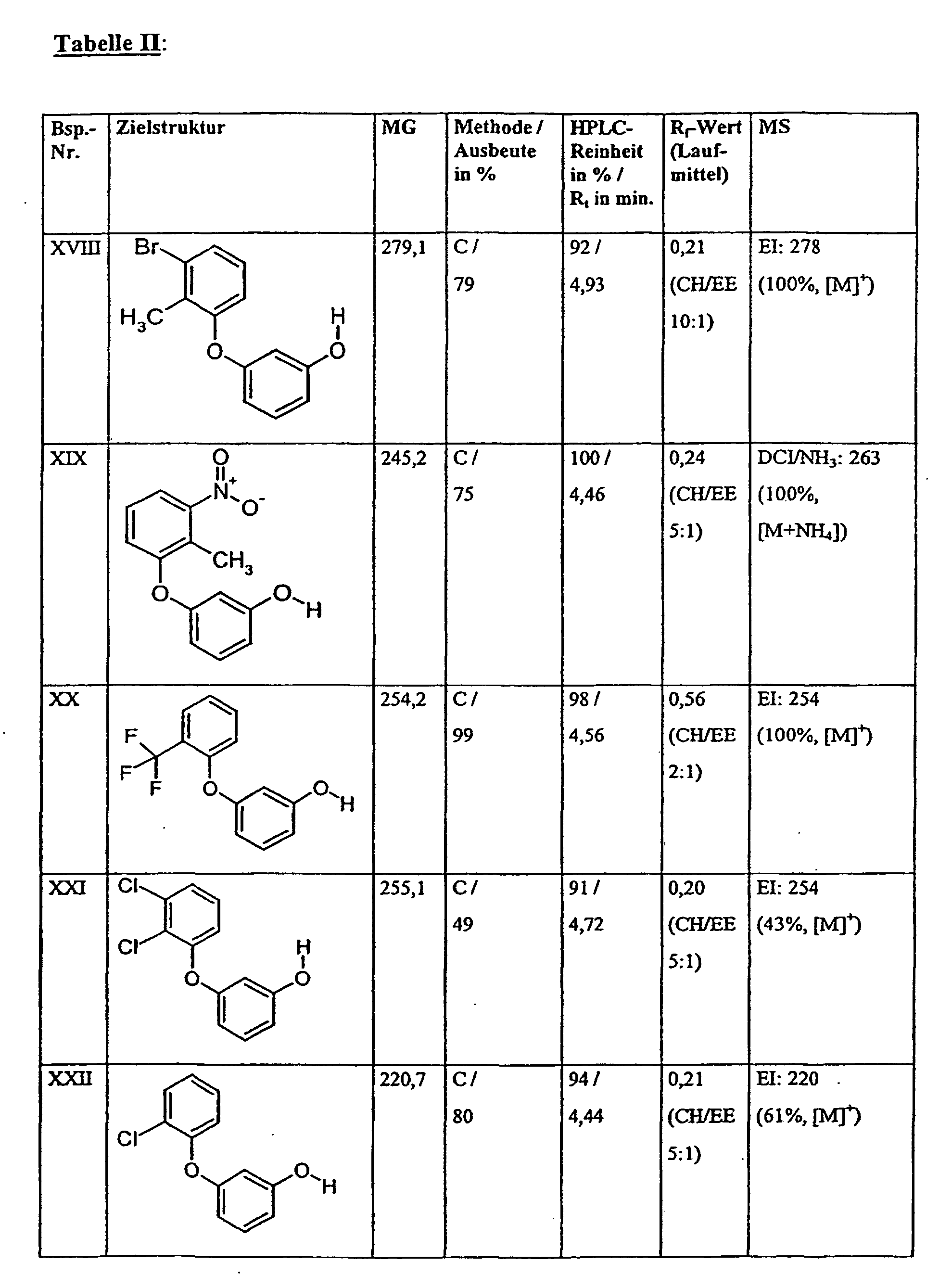

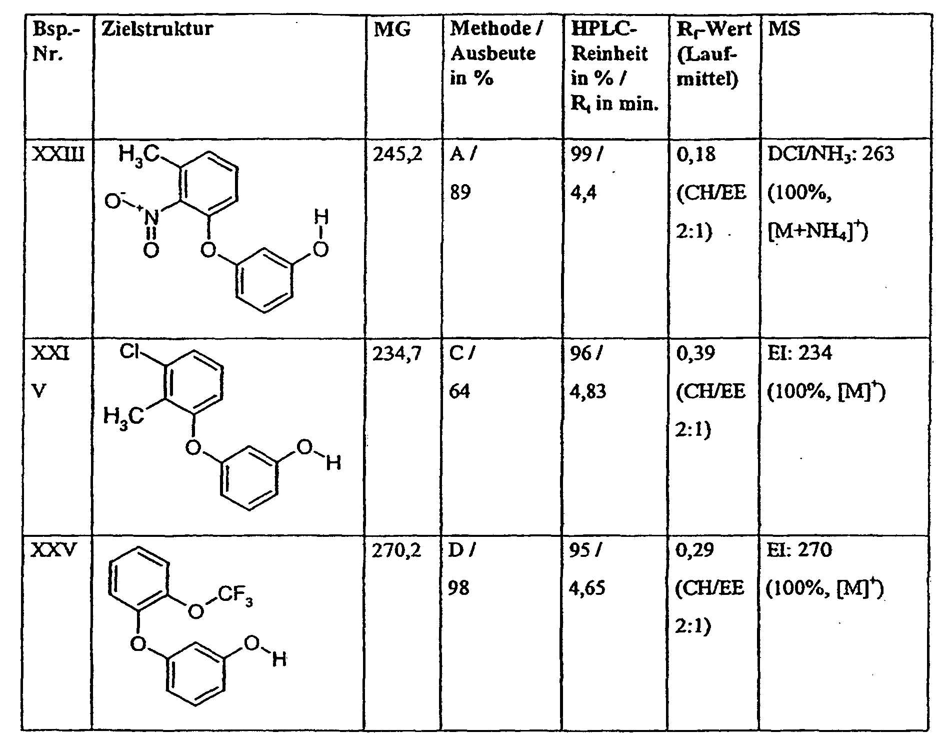

### Beispiel XXVI

### 3-[2-Chlor-3-(trifluormethyl)phenoxy]-anilin

Unter Argon werden 630 mg (1,98 mmol) 1-[2-Chlor-3-(trifluormethyl)phenoxy]-3-nitrobenzol mit 625 mg (9,09 mmol) Ammoniumformiat und 31,5 mg 10 % Palladium/Kohle-Katalysator in 7 ml Methanol vorgelegt. Das Gemisch wird für zwei Stunden zum Rückfluß erhitzt. Nach Abkühlen wird über Kieselgur filtriert, mit Methanol nachgewaschen und das Filtrat eingeengt. Man nimmt in Dichlormethan wieder auf, extrahiert dreimal mit Wasser, trocknet die organischen Phasen über Magnesiumsulfat und engt wieder ein. Chromatographische Reinigung an Kieselgel im Laufinittel Cyclohexan/Ethylacetat 6:1 ergibt 458 mg (72 %, HPLC-Reinheit 90 %) der Zielverbindung.
R_{f}: 0,48 (Cyclohexan/Ethylacetat 1:1)
MS (ESI): 288 (22 %, [M+H]⁺)
HPLC: Retentionszeit = 4,41 min
¹H-NMR (300 MHz, DMSO-d₆): δ /ppm = 5,28 (s, 2H), 6,11-6,19 (m, 2H), 6,38 (ddd, J = (Hz, 2Hz, 1Hz, 1H), 7,03 (t, J = 8 Hz, 1H), 7,33 (dd, J = 8Hz, 1Hz, 1H), 7,54 (t, J = 8Hz, 1H), 7,63 (dd, J = 8Hz, 1Hz).

### Beispiel XXVII

### 1-[2-Cyano-3-(trifluormethyl)phenoxy]-3-hydroxybenzol

### (Diphenylethersynthese - Methode E)

Resorcinol [44,0 g (0,4 mol) wird in 170 ml N-Methyl-pyrrolidon teilweise gelöst, mit Kaliumhydroxid [mind. 85 %ig; 34,5 g (0,52 mol)] und dann mit 2-Chlor-6-(trifluormethyl)benzonitril [20,5 g (0,1 mol)] versetzt. Das Gemisch wird 2,5 h bei 60-65°C gerührt. Nach Zugabe von 300 ml Toluol und 400 ml Wasser wird die wässrige Phase abgetrennt und noch einmal mit 300 ml Toluol extrahiert. Die vereinten organischen Phasen werden über MgSO₄ getrocknet und nach Filtration eingeengt. Digerieren des öligen Rückstandes mit 150 ml Wasser, Filtration und Trocknen ergibt leicht bräunliche Kristalle.
Ausbeute: 22 g (79 % d.Th.; vgl. Beispiel XV)

### Herstellungsbeispiele

### Beispiel 1

### 3-[2-Cyano-3-(trifluormethyl)phenoxy]phenyl 4,4,4-trifluor-1-butansulfonat.

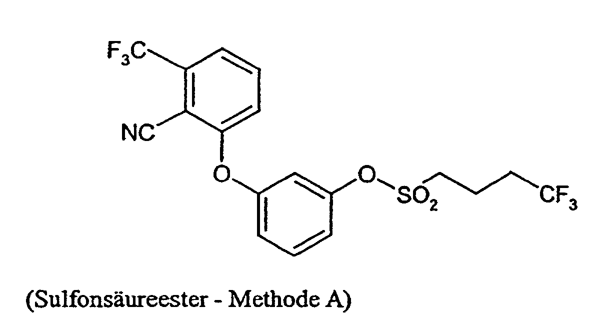

Unter Argon werden 7,70 g (27,6 mmol) 3-[2-Cyano-3-(trifluormethyl)phenoxy]phenol in 60 ml Dichlormethan gelöst, anschließend die Lösung mit 4,32 g (13,1 mmol) Tetrabutylammoniumbromid und 3,95 ml 45 %iger NaOH versetzt. Bei 0°C werden 6,64 g (31,5 mmol) 4,4,4-Trifluorbuitan-1-sulfonsäurechlorid gelöst in 20 ml Dichlormethan in einem Guß zugegeben. Man rührt die sich gelb bis orange färbende Lösung 1h nach. Nach anschließendem Verdünnen mit Wasser wird dreimal mit Dichlormethan extrahiert. Die vereinigten organischen Phasen werden mit Kochsalzlösung gewaschen und über Magnesiumsulfat getrocknet. Reinigung erfolgt durch Flashchromatographie an 360 g Kieselgel in einem stufenweisen Laufmittelgradienten von Cyclohexan/Dichlormethan 1:1 bis 1:4. Einrotieren hinterläßt einen öligen Rückstand, der durch Zugabe von Pentan zur Kristallisation gebracht wird.
Ausbeute: 1. Fraktion 9,21 g (74 %, HPLC-Reinheit 100 %)
2. Fraktion 2,29 g (18 %, HPLC-Reinheit 97 %)
R_{f}: 0,56 (CH₂Cl₂)
Schmelzpunkt: 60-61°C
MS (ESI): 454 ([M+H]⁺)
HPLC: Retentionszeit = 5,08 min
¹H-NMR (300 MHz, CDCl₃): δ /ppm = 2,2-2,5 (m, 4H), 3.39 (t, J = 7Hz, 2H), 7,0-7,3 (m, 4H), 7,50 (t, J = 8Hz, 1H), 7,53 (d, J = 8Hz, 1H), 7,64 (d, J = 8Hz).

### Beispiel 2

### 3-(3-Methyl-2-nitro-phenoxy)phenyl n-Pentansulfonat.

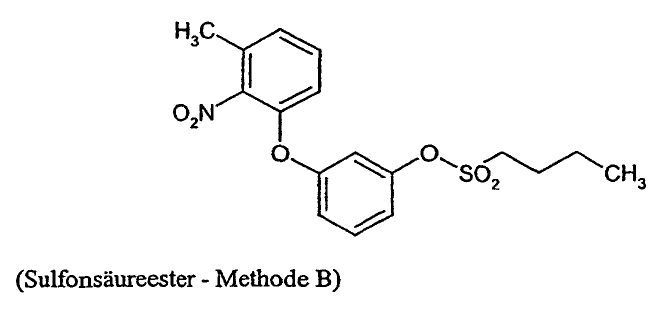

Zu 200 mg (0,82 mmol) 3-(3-Methyl-2-nitrophenoxy)-phenol werden bei Raumtemperatur in 5 ml Dichlormethan zunächst 1 ml 40 %ige Tetrabutylammoniumhydroxidlösung gegeben, dann nach 5 min Rühren 153 mg (0,90 mmol) n-Pentansulfonsäurechlorid. Nach 1,5 h Rühren wird mit 0,5 ml 10 %iger NaHCO₃-Lösung versetzt, das Gemisch über eine 3 g-Extrelut-Kartusche (Merck Darmstadt, Best-Nr. 115095) filtriert und die Kartusche mehrmals mit Dichlormethan nachgewaschen. Chromatographische Reinigung an Kieselgel im Laufinittel Cyclohexan/Ethylacetat 30:1 ergibt 255 mg (82 %, HPLC-Reinheit 99 %) der Zielverbindung.
R_{f}: 0,35 (Cyclohexan/Ethylacetat 2:1)
MS (ESI): 380 (100%, [M+H]⁺)
HPLC: Retentionszeit = 5,29 min
¹H-NMR (300 MHz, CDCl₃): δ /ppm = 0,93 (t, J = 7Hz, 3H), 1,30-1,51 (m, 4H), 1,89-2,02 (m, 2H), 2,39 (s, 3H), 3,18-3,28 (m, 2H); 6,85-7,42 (m, 7H).

### Beispiel 3

### N-{3-[2-Chlor-3-(trifluormethyl)phenoxy]phenyl}-4,4,4-trifluorbutan-1-sulfonsäureamid

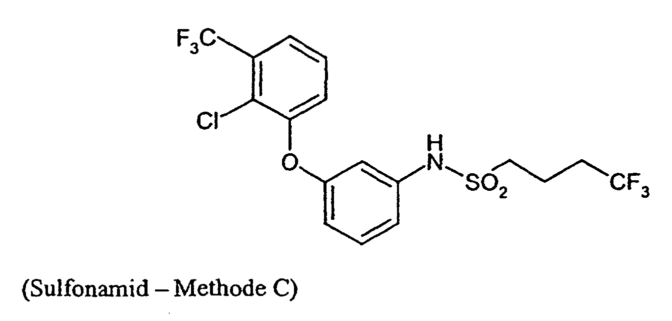

Unter Argon werden 100 mg (0,35 mmol) 3-[2-Chlor-3-(trifluormethyl)phenoxylanilin in 1 ml Dichlormethan vorgelegt. Man gibt 106 mg (1,04 mmol) Triethylamin und 77 mg (0,37 mmol) 4,4,4-Trifluorbutan-1-sulfonsäurechlorid, gelöst in 1ml Dichlormethan, zu und rührt bei Raumtemperatur. Nach vier Tagen gibt man weitere 0,3 Äquivalente 4,4,4-Trifluorbutansulfonsäurechlorid zu und rührt weitere drei Tage. Anschließend wird der Ansatz dreimal mit 2N Salzsäure und einmal mit gesättigter Kochsalzlösung extrahiert. Die organische Phase wird über Magnesiumsulfat getrocknet und im Vakuum eingeengt. Chromatographische Reinigung an Kieselgel im Laufmittel Dichlormethan/Cyclohexan 7:2 ergibt 96 mg (54 %, HPLC-Reinheit 90 %) der Zielverbindung.
R_{f}: 0,33 (Cyclohexan/Ethylacetat 2:1)
MS (DCI/NH₃): 479 (100%, [M+NH₄]⁺)
HPLC: Retentionszeit = 8,34 min
¹H-NMR (300 MHz, DMSO-d₆): δ /ppm = 1,80 -1,93 (m, 2H), 2,31 - 2,50 (m, 2H), 3,25 (t, J = 8Hz, 2H), 6,75 (dd, J = 8Hz, 2Hz, 1H), 6,85 (t, J = 2Hz, 1H), 7,03 (dd, J = 8Hz, 1Hz), 7,38 (t, J= 8Hz, 1H), 7,43 (dd, J = 8Hz,1Hz, 1H), 7,58 (t, J = 8Hz, 1H), 7,72 (dd, J = 8Hz, 1Hz, 1H), 10,04 (s, 1H).

Die folgenden Beispiele 4 bis 24 werden auf analoge Weise entsprechend den Herstellungsbeispiel-Verfahrensmethoden A, B oder C aus den entsprechenden Ausgangsverbindungen hergestellt:

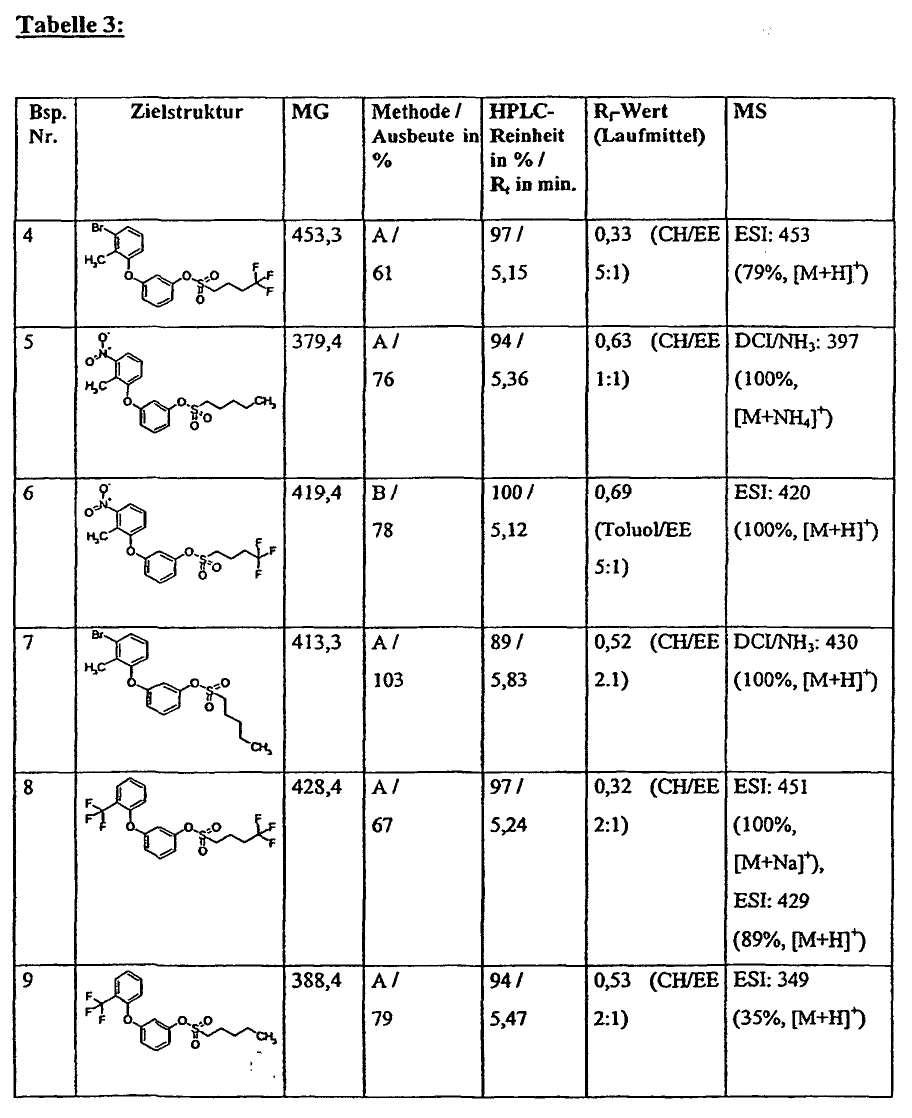

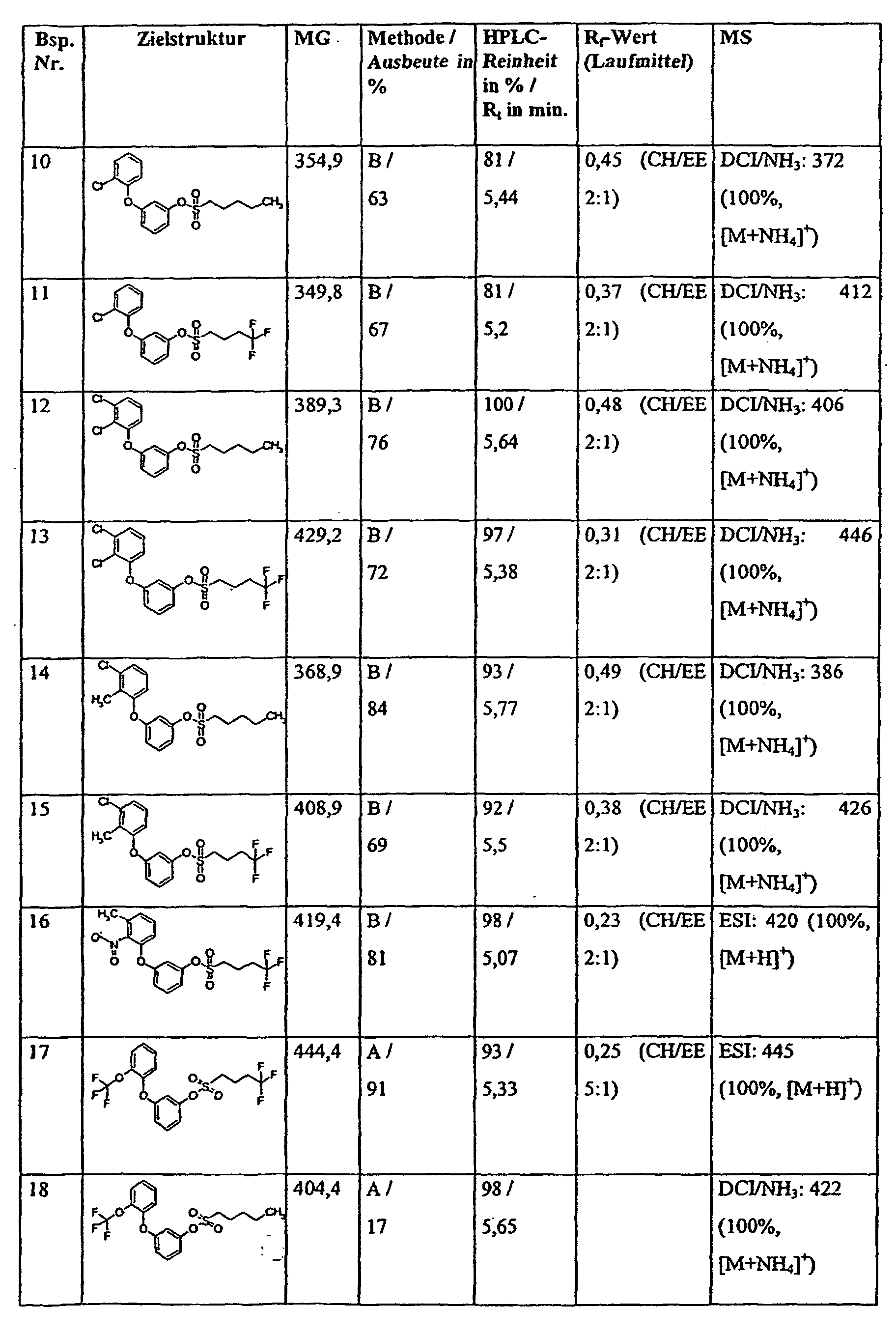

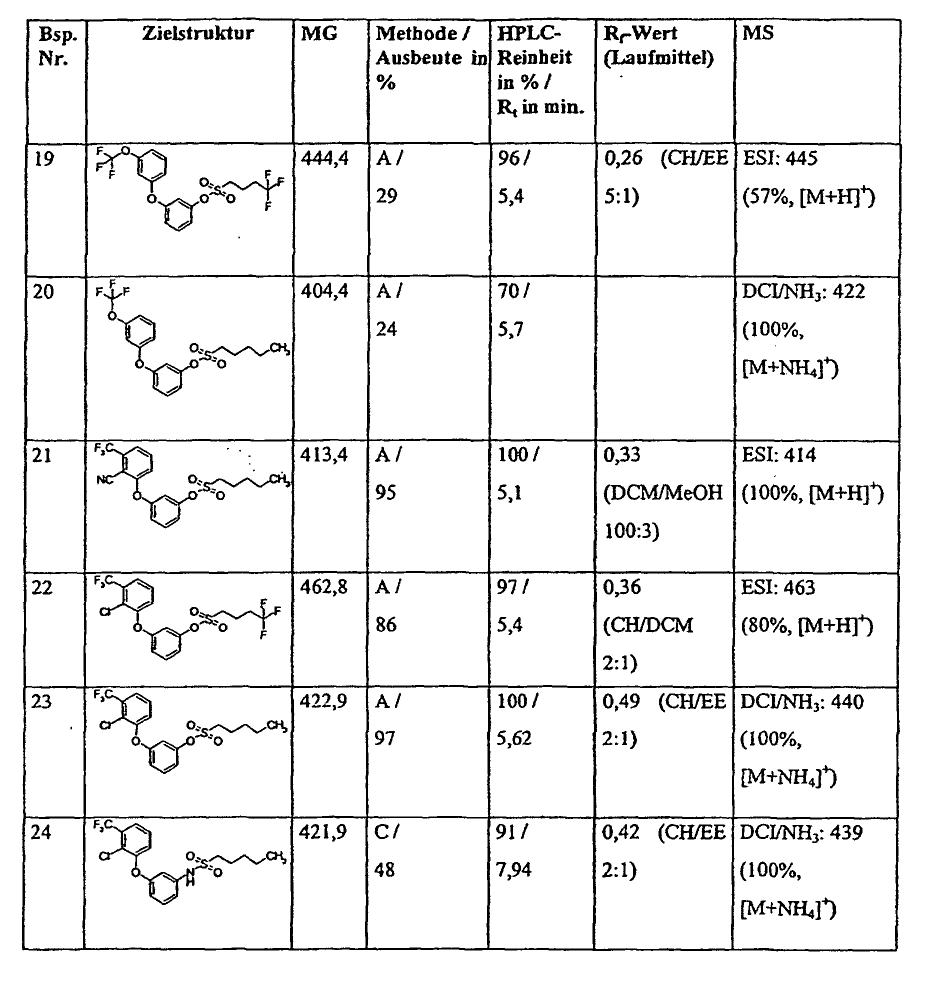

Die oben genannten Beispiele zeigen folgende ¹H-NMR-spektroskopischen Daten:

**Tabelle 4:**

| **Beispiel** | ^{**1**}**H-NMR** (300 MHz): δ/ppm |
|---|---|
| 17 | CDCl₃; 2,17-2,42 (m, 4H); 3,33 (t, J=7Hz, 2H); 6,88 (t, J=2Hz; 1H) 6,92-6,95 (m, 1H); 7,01-7,05 (ddd, H=8 Hz, 2Hz, 0,5Hz; 1H); 7,09 (dd, J=8Hz, 2Hz, 1H); 7,18-7,39 (m, 4H). |
| 21 | DMSO-d₆; 0,86 (t, J=7Hz, 3H); 1,24-1,43 (m, 4H); 1,80 (quin, 2H); 3,56 (t, J=7,5Hz, 2H); 7,29-7,38 (m, 4H); 7,61 (t, J=8Hz, 1H); 7,77 (d, H=7,5Hz, 1H); 7,88 (t, J=8Hz, 1H). |
| 22 | DMSO-d₆; 2,02 (quin, 2H); 2,46 (m, 2H); 3,69 (t, J=7,5Hz, 2H); 7,02-7,08 (m, 3H); 7,17-7,20 (dd, J=8Hz, 2Hz, 1H); 7,48-7,63 (m, 3H); 7,72-7,75 (dd, J=8Hz, 1Hz, 1H). |
| 23 | DMSO-d₆; 0,86 (t, J=7Hz, 3H); 1,33 (m, 4H); 1,78 (quin, 2H); 3,52 (t, J=7,5Hz, 2H); 7,02-7,06 (m, 2H); 7,15-7,18 (m, 1H); 7,48-7,64 (m, 3H); 7,72-7,75 (dd, J=8*Hz, 1Hz, 1H). |

## Patentansprüche

1. Verbindungen der allgemeinen Formel (I), 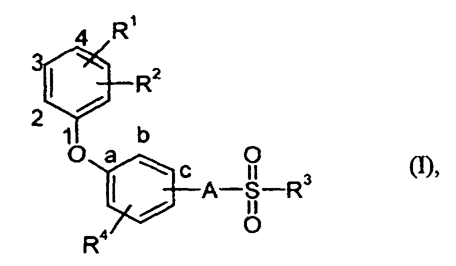 worin
R¹ Wasserstoff, (C₁-C₄)-Alkyl, Halogen, Trifluormethyl, Trifluormethoxy, Cyano oder Nitro bedeutet,
R² Halogen, Trifluormethyl, Trifluormethoxy, Cyano oder Nitro bedeutet,
R³ (C₄-C₇)-Alkyl bedeutet, das ein- oder mehrfach durch Fluor oder Chlor substituiert sein kann,
R⁴ Wasserstoff oder Halogen bedeutet, und
A Sauerstoff oder NH bedeutet,
sowie deren Salze und Ammoniumverbindungen.

2. Verbindungen nach Anspruch 1,
wobei
R¹ Wasserstoff, Fluor, Chlor, Methyl, Trifluormethyl, Trifluormethoxy, Cyano oder Nitro bedeutet,
R² Fluor, Trifluormethyl, Trifluormethoxy, Cyano oder Nitro bedeutet,
R³ n-Butyl, n-Pentyl, 4,4,4-Trifluorbut-1-yl oder 5,5,5-Trifluorpent-1-yl bedeutet,
R⁴ Wasserstoff bedeutet, und
A Sauerstoff bedeutet,
sowie deren Salze.

3. Verbindungen nach Anspruch 1 oder 2,
wobei
R¹, R², R³, R⁴ und A die in Anspruch 1 oder 2 angegebene Bedeutung haben, und
ein Wasserstoffatom in Position 4 des mit R¹ und R² substituierten Phenylrings steht,
sowie deren Salze und Ammoniumverbindungen.

4. Verbindungen nach Anspruch 1 oder 2,
wobei
R¹, R², R³, R⁴ und A die in Anspruch 1 oder 2 angegebene Bedeutung haben, und
R¹ und R² die Positionen 2 und 3 des Phenylrings besetzen,
sowie deren Salze und Ammoniumverbindungen.

5. Verbindungen nach einem der Ansprüche 1 bis 3,
wobei
R^{1,} R², R³, R⁴ und A die in einem der Ansprüche 1 bis 4 angegebene Bedeutung haben, und
A in Position c des Benzolrests steht,
sowie deren Salze und Ammoniumverbindungen.

6. Verfahren zur Herstellung von Verbindungen nach Anspruch 1, **dadurch gekennzeichnet, dass** man eine Verbindung der allgemeinen Formel (II) 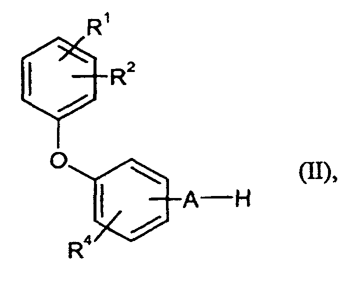 in welcher R¹, R², R⁴ und A die in Anspruch 1 angegebene Bedeutung haben,
in einem inerten Lösemittel in Gegenwart einer geeigneten Base und gegebenenfalls in Gegenwart eines Phasentransfer-Katalysators mit einer Verbindung der allgemeinen Formel (III)
X¹-SO₂-R³ (III),
in welcher
X¹ für eine geeignete Abgangsgruppe steht, und
R³ die oben angegebene Bedeutung hat,
umsetzt.

7. Verbindungen nach einem der Ansprüche 1 bis 5 zur Behandlung und/oder Prophylaxe von Krankheiten.

8. Arzneimittel enthaltend mindestens eine der Verbindungen nach einem der Ansprüche 1 bis 5 in Zusammenmischung mit mindestens einen pharmazeutisch verträglichen, im wesentlichen nichtgiftigen Träger oder Exzipienten.

9. Verwendung von Verbindungen nach einem der Ansprüche 1 bis 5 zur Herstellung eines Arzneimittels zur Behandlung und/oder Prophylaxe von Schmerzzuständen und/oder neurodegenerativen Erkrankungen.

10. Verwendung von Verbindungen nach einem der Ansprüche 1 bis 5 zur Herstellung eines Arzneimittels zur Behandlung und/oder Prophylaxe der Parkinsonschen Krankheit.

## Claims

1. Compounds of the general formula (I), 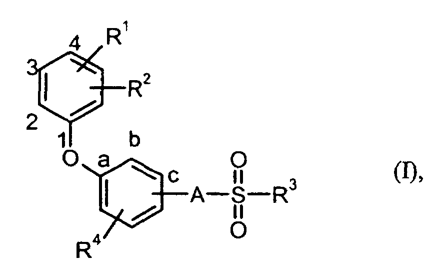 in which
R¹ denotes hydrogen, C₁-C₄-alkyl, halogen, trifluoromethyl, trifluoromethoxy, cyano or nitro,
R² denotes halogen, trifluoromethyl, trifluoromethoxy, cyano or nitro,
R³ denotes C₄-C₇-alkyl which may be substituted one or more times by fluorine or chlorine,
R⁴ denotes hydrogen or halogen, and
A denotes oxygen or NH,
and the salts and ammonium compounds thereof.

2. Compounds according to Claim 1,
where
R¹ denotes hydrogen, fluorine, chlorine, methyl, trifluoromethyl, trifluoromethoxy, cyano or nitro,
R² denotes fluorine, trifluoromethyl, trifluoromethoxy, cyano or nitro,
R³ denotes n-butyl, n-pentyl, 4,4,4-trifluorobut-1-yl or 5,5,5-trifluoropent-1-yl,
R⁴ denotes hydrogen, and
A denotes oxygen,
and the salts thereof.

3. Compounds according to Claim 1 or 2,
where
R¹, R², R³, R⁴ and A have the meaning stated in Claim 1 or 2, and
there is a hydrogen atom in position 4 of the phenyl ring substituted by R¹ and R²,
and the salts and ammonium compounds thereof.

4. Compounds according to Claim 1 or 2,
where
R¹, R², R³, R⁴ and A have the meaning stated in Claim 1 or 2, and
R¹ and R² occupy positions 2 and 3 on the phenyl ring,
and the salts and ammonium compounds thereof.

5. Compounds according to any of Claims 1 to 3,
where
R¹, R², R³, R⁴ and A have the meaning stated in any of Claims 1 to 4, and
A is in position c of the benzene radical,
and the salts and ammonium compounds thereof.

6. Process for preparing compounds according to Claim 1, **characterized in that** a compound of the general formula (II) 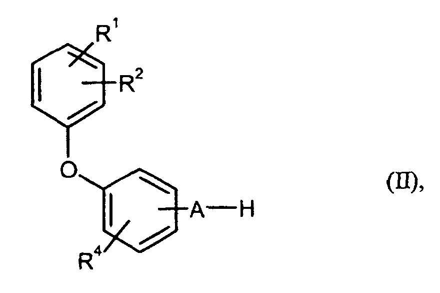 in which R¹, R², R⁴ and A have the meaning stated in Claim 1, is reacted in an inert solvent in the presence of a suitable base and, where appropriate, in the presence of a phase-transfer catalyst with a compound of the general formula (III)
X¹- SO₂ - R³ (III),
in which
X¹ represents a suitable leaving group, and
R³ has the abovementioned meaning.

7. Compounds according to any of Claims 1 to 5 for the treatment and/or prophylaxis of diseases.

8. Medicaments containing at least one of the compounds according to any of Claims 1 to 5 mixed with at least one pharmaceutically suitable essentially nontoxic carrier or excipient.

9. Use of compounds according to any of Claims 1 to 5 for producing a medicament for the treatment and/or prophylaxis of states of pain and/or neurodegenerative disorders.

10. Use of compounds according to any of Claims 1 to 5 for producing a medicament for the treatment and/or prophylaxis of Parkinson's disease.

## Revendications

1. Composés de formule générale (I), 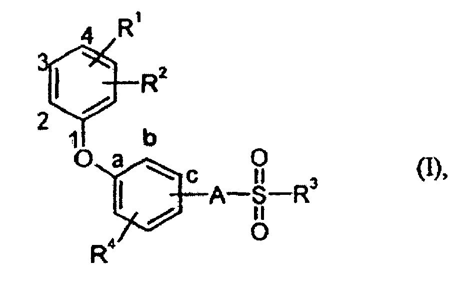 dans laquelle
R¹ représente l'hydrogène, un groupe alkyle en C₁ à C₄, un halogène, un groupe trifluorométhyle, trifluorométhoxy, cyano ou nitro,
R² représente un halogène, un groupe trifluorométhyle, trifluorométhoxy, cyano ou nitro,
R³ représente un groupe alkyle en C₄ à C₇, qui peut être substitué une ou plusieurs fois par du fluor ou du chlore,
R⁴ représente l'hydrogène ou un halogène, et
A représente l'oxygène ou le groupe NH,
ainsi que leurs sels et leurs composés d'ammonium.

2. Composés suivant la revendication 1,
dans lesquels
R¹ représente l'hydrogène, le fluor, le chlore, un groupe méthyle, trifluorométhyle, trifluorométhoxy, cyano ou nitro,
R² représente le fluor, un groupe trifluorométhyle, trifluorométhoxy, cyano ou nitro,
R³ représente un groupe n-butyle, n-pentyle, 4,4,4-trifluorobut-1-yle ou 5,5,5-trifluoropent-1-yle,
R⁴ représente l'hydrogène, et
A représente l'oxygène,
ainsi que leurs sels.

3. Composés suivant la revendication 1 ou 2,
dans lesquels
R¹, R², R³, R⁴ et A ont la définition indiquée dans la revendication 1 ou 2,
et
un atome d'hydrogène se trouve en position 4 du noyau phényle substitué avec R¹ et R²,
ainsi que leurs sels et leurs composés d'ammonium.

4. Composés suivant la revendication 1 ou 2,
dans lesquels
R¹, R², R³, R⁴ et A ont la définition indiquée dans la revendication 2,
et
R¹ et R² occupent les positions 2 et 3 du noyau phényle, ainsi que leurs sels et leurs composés d'ammonium.

5. Composés suivant l'une des revendications 1 à 3,
dans lesquels
R¹, R², R³, R⁴ et A ont la définition indiquée dans l'une des revendications 1 à 4, et
A occupe la position c du reste benzène,
ainsi que leurs sels et leurs composés d'ammonium.

6. Procédé de production de composés suivant la revendication 1, **caractérisé en ce qu'**on fait réagir un composé de formule générale (II) 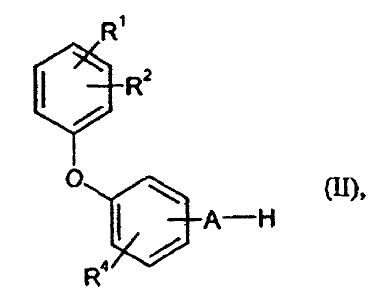 dans laquelle R¹, R², R⁴ et A ont la définition indiquée dans la revendication 1, dans un solvant inerte en présence d'une base convenable et, le cas échéant, en présence d'un catalyseur de transfert de phase, avec un composé de formule générale (III)
X¹ - SO₂ - R³ (III),
dans laquelle
X¹ représente un groupe partant convenable, et
R³ a la définition indiquée ci-dessus.

7. Composés suivant l'une des revendications 1 à 5, destinés au traitement et/ou à la prophylaxie de maladies.

8. Médicament contenant au moins l'un des composés suivant l'une des revendications 1 à 5 en mélange avec au moins un support ou excipient essentiellement non toxique, acceptable du point de vue pharmaceutique.

9. Utilisation de composés suivant l'une des revendications 1 à 5 pour la préparation d'un médicament destiné au traitement et/ou à la prophylaxie d'états douloureux et/ou de maladies neurodégénératives.

10. Utilisation de composés suivant l'une des revendications 1 à 5 pour la préparation d'un médicament destiné au traitement et/ou à la prophylaxie de la maladie de Parkinson.
